# EUROPEAN PATENT APPLICATION

(11) **EP 4 512 894 A1**
(43) Date of publication of application: **26.02.2025**
(21) Application number: 23192560.3
(22) Date of filing: 22.08.2023
(51) Int. Cl.: C12N 9/38

(54) **FRUCTANASE VARIANTS**

(71) Applicant: AB Enzymes Finland Oy, 05200 Rajamäki (FI)
(72) Inventor: JUNTUNEN, Kari, 05200 Rajamäki (FI); PURANEN, Terhi, 05200 Rajamäki (FI); ALAPURANEN, Marika, 05200 Rajamäki (FI); OJA, Merja, 05200 Rajamäki (FI); FISCHER, Felix, 64293 Darmstadt (DE); BESENMATTER, Werner, 64293 Darmstadt (DE)
(74) Representative: Espatent Oy

(57) **Abstract**

A variant polypeptide of fructanase, a fusion protein and an enzyme composition comprising said variant polypeptide, recombinant host cell producing the variant polypeptide, method for manufacturing the variant polypeptide, a use of the variant polypeptide to degrade and modify fructan containing material, and a premix for baking comprising the variant polypeptide.

## Description

### TECHNICAL FIELD

The present disclosure generally relates to the field of enzyme technology. The disclosure relates particularly, though not exclusively, to fructanase variants having an increased productivity and/or fructan degradation activity. The present fructanase variants are useful in various applications where degradation of fructan is desired, such as in plant-based and baked products.

### BACKGROUND

This section illustrates useful background information without admission of any technique described herein representative of the state of the art.

Fructans are comprised of fructose residues, normally with a terminal sucrose unit (i.e. a glucose-fructose disaccharide). The linkage position of the fructose residues determines the type of the fructan. The basic types of single-linkage fructans are inulin and levan (or phlein). Additionally, there exists a mixed-linkage fructan called graminan. Common sources of fructans include plants, such as cereals, vegetables, fruits and nuts.

Several fructan degrading enzymes, i.e. fructanases, are known in the art. Fructanases are fructosidases or exo-beta-D-fructosidases, which are enzymes that catalyze the hydrolysis of a fructan. More specifically, fructanase catalyzes the hydrolysis of terminal, non-reducing (2->1)- and (2->6)-linked beta-D-fructofuranose residues in fructans. Substrates of fructanase include fructans such as inulin and levan, and sucrose.

The use of fructanases for degradation of fructans in plant-based materials have been described for various purposes. Fructanases can be added to hydrolyze fructans, for example, to achieve increased softness of the food, or to bring additional sweetness to the food. In WO2017220864 fructanases are used to degrade fructans, to obtain foodstuff suitable for a diet low in fermentable carbohydrates, known as FODMAP.

However, fructanase enzymes are often produced in insufficient quantities, the production yields thereby not being economically feasible. Moreover, the activity of the known fructanase enzymes may be low and inadequate to be used, for example with foodstuff, to hydrolyze fructans efficiently. Therefore, new fructanase enzymes are needed, having higher production level and specific fructan degrading activity.

It is an object of the present disclosure to provide fructanase variants that exhibit fructanase activity and that have improved properties that allow their use in industrial processes. Yet another object of the present disclosure is to provide fructanase variants that can be used in enzyme compositions for fructan degradation in foodstuff, such as in baked products. Yet another object of the present disclosure is to provide fructanase variants that can be used for fructan degradation in feedstuff and feed applications, and in detergents.

### SUMMARY

The appended claims define the scope of protection. Any examples and technical descriptions of apparatuses, products and/or methods in the description and/or drawings not covered by the claims are presented not as embodiments of the invention but as background art or examples useful for understanding the invention.

The present disclosure provides a novel variant polypeptide with fructanase activity, being able to efficiently degrade fructan. The disclosure relates particularly, though not exclusively, to fructanase variants having an increased activity and/or productivity.

According to a first aspect there is provided a variant polypeptide of a wild type fructanase, having:
fructanase activity;
an amino acid sequence having at least 65 % amino acid sequence identity with the amino acids 374 - 850 of SEQ ID NO: 12; and
   at least one substitution, insertion or deletion of an amino acid, the at least one substitution, insertion or deletion preventing N-glycosylation of at least one amino acid when expressed in a host cell capable of N-glycosylation, and/or
   at least 50 amino acids deleted when compared to the wild type parent fructanase of the variant polypeptide.

It was surprisingly found and shown in the examples below that by preventing N-glycosylation of at least one amino acid of the variant polypeptide in host cells capable of N-glycosylation via at least one substitution, insertion or deletion of an amino acid, and/or by deleting at least 50 amino acids from the wild type parent fructanase of the variant polypeptide which has at least 65 % amino acid sequence identity with the amino acids 374 - 850 of SEQ ID NO: 12, the activity and/or productivity of the variant polypeptide in production hosts is significantly increased. The variant polypeptide of first aspect is thus beneficial, as it has increased fructanase productivity and/or improved fructan degrading performance, when compared to a polypeptide having the sequence SEQ ID NO: 12 or when compared to the wild type parent fructanase of the variant polypeptide.

According to a second aspect there is provided a fusion protein, comprising the amino acid sequence of the variant polypeptide according to the first aspect, and at least one:
an amino acid sequence providing a secretory signal sequence;
an amino acid sequence which facilitates purification, such as an affinity tag or His-tag;
an amino acid sequence controlling and enhancing the production of the fusion protein, such as a carrier polypeptide;
an amino acid sequence encoding a protease cleavage site;
a linker sequence;
an amino acid sequence having an enzyme activity; and/ or
an amino acid sequence providing for the fusion protein with binding affinity, such as a carbohydrate binding moiety.

According to a third aspect there is provided an enzyme composition comprising the variant polypeptide of the first aspect or the fusion protein of the second aspect.

The present fructanase variant, the fusion protein and the enzyme composition are useful in various applications where modification, degradation or partial degradation of fructan is desired, such as in plant and grain-based products, particularly in baked products.

According to a fourth aspect there is provided a recombinant host cell comprising genetic elements that allow producing at least one variant polypeptide of the first aspect, or the fusion protein of the second aspect.

The recombinant host cell is beneficial, as it has increased fructanase productivity, when compared to a host cell comprising genetic elements that allow producing the polypeptide having the sequence SEQ ID NO: 12, or when compared to a host cell comprising genetic elements that allow producing the polypeptide of the wild type parent fructanase of the variant polypeptide. It was further surprisingly found that production of the fructanase variant polypeptide in production host cells, significantly increases the productivity of the fructanase polypeptide without negatively affecting its ability to degrade fructans.

According to a fifth aspect there is provided a use of the variant polypeptide of the first aspect, and/or the fusion protein of the second aspect and/or the enzyme composition of the third aspect, for degradation of fructan in plant-based material.

According to a sixth aspect there is provided a method of manufacturing the variant polypeptide of the first aspect or the fusion protein of the second aspect, comprising: cultivating the recombinant host cell of the fourth aspect in conditions allowing production of the at least one variant polypeptide of the first aspect, or the fusion protein of the second aspect; and optionally recovering the variant polypeptide or the fusion protein.

According to a seventh aspect there is provided a premix for baking, comprising the variant polypeptide of the first aspect, and/or the fusion protein of the second aspect and/or the enzyme composition of the third aspect.

Different non-binding example aspects and embodiments have been illustrated in the foregoing. The embodiments in the foregoing are used merely to explain selected aspects or steps that may be utilized in different implementations. Some embodiments may be presented only with reference to certain example aspects. It should be appreciated that corresponding embodiments may apply to other example aspects as well.

### BRIEF DESCRIPTION OF THE FIGURES

Some example embodiments will be described with reference to the accompanying figures, in which:

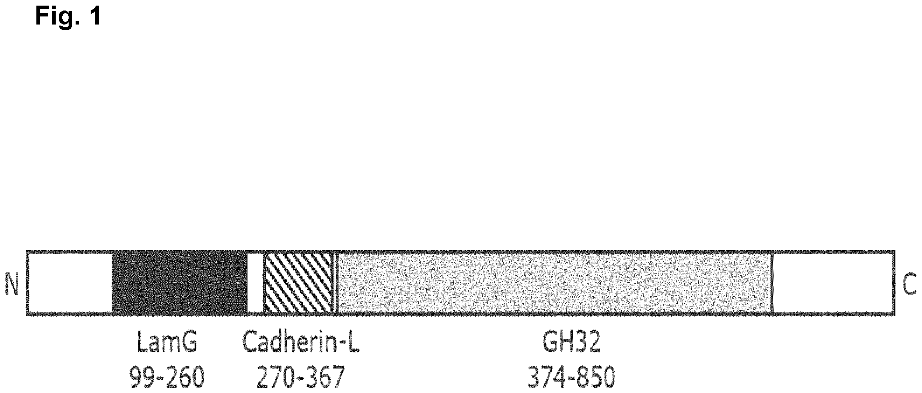

- Fig. 1: Schematically shows a picture of LFM37 fructanase polypeptide according to SEQ ID NO:12, comprising multiple putative domains including a GH32 domain amino acids 374 - 850, according to an example embodiment.

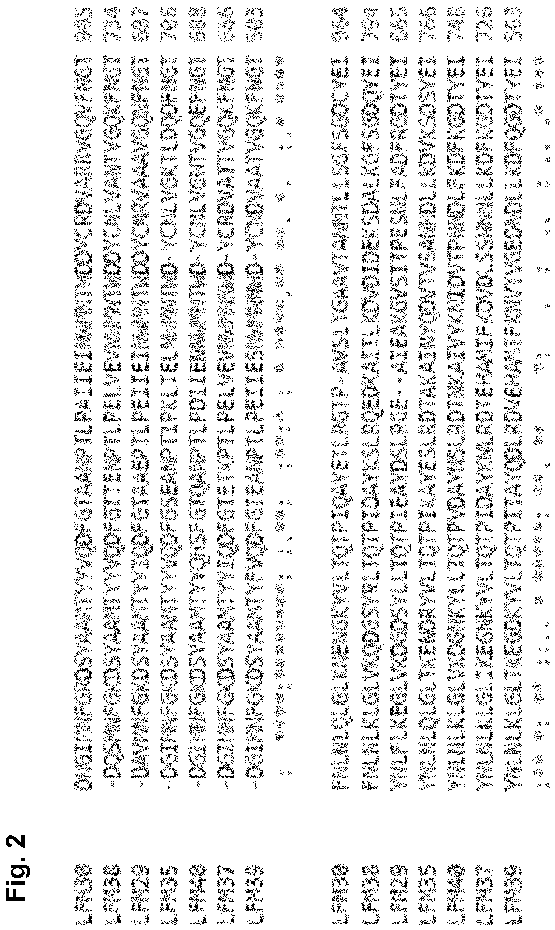

- Fig. 2: Shows an alignment of the wild type fructanase amino acid sequences LFM30, LFM38, LFM29, LFM35, LFM40, LFM37 and LFM39 having a high sequence identity with each other upstream and downstream of the respective glycosylation site of conserved asparagine at amino acid position corresponding to the N664 of wild type LFM37 (SEQ ID NO: 12).

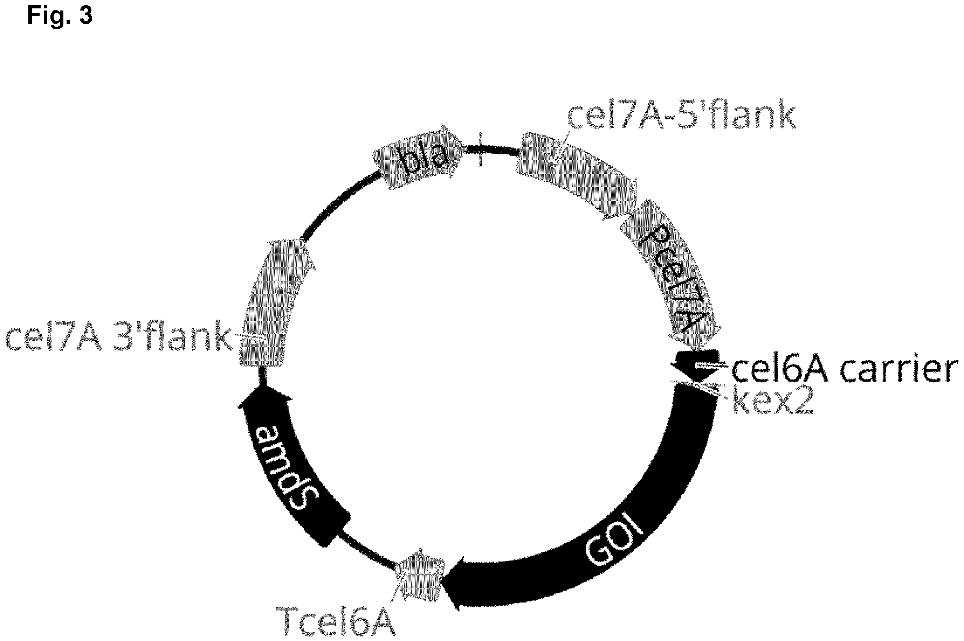

- Fig. 3: Schematically shows a picture of the expression plasmid used in the transformation of *T. reesei* for expression of fructanase (wildtype and variants) genes (= GOI, Gene of interest). Expression of the recombinant genes in the host cell was controlled using the following genetic elements: *T. reesei* cel7A promoter (Pcel7A) for transcription initiation, and *T. reesei* cel6A (Tcel6A) terminator for transcription termination. *T. reesei* cel6A carrier encoding the cel6A CBM and linker region was used instead of the native fructanase signal sequence with Kex2 protease cleavage site (kex2) included between the encoded carrier polypeptide and the fructanase. The synthetic amdS marker gene encoding for acetamidase was included for selection of the transformants and *T. reesei* cel7A 5'- and 3'-flanking regions were used for optional targeting of the expression construction to cel7A locus. The picture was generated using Geneious Prime 2021 created by Biomatters.

### SEQUENCE LISTING

SEQ ID NO: 1 **LFM35**, Amino acid sequence of a full-length *Streptococcus sobrinus W1703* fructosidase LFRU without signal peptide.
SEQ ID NO: 2 **LFM35GS,** Amino acid sequence of a truncated *Streptococcus sobrinus W1703* fructosidase LFRU without signal peptide, contains the amino acids 1-1152 of the full-length wild-type fructosidase, comprising a mutation T706A.
SEQ ID NO: 3 **LFM38,** Amino acid sequence of a full-length *Streptococcus parvulum* fructosidase LFRU without signal peptide.
SEQ ID NO: 4 **LFM38GS,** Amino acid sequence of a truncated *Streptococcus parvulum* fructosidase LFRU without signal peptide, contains the amino acids 151-1181 of the full-length wild-type fructosidase, comprising a mutation T734A.
SEQ ID NO: 5 **LFM21**, Amino acid sequence of a full-length *Clostridium pasteurianum DSM* 525 fructosidase LFRU without signal peptide.
SEQ ID NO: 6 **LFM21G**, Amino acid sequence of a full-length *Clostridium pasteurianum DSM* 525 fructosidase LFRU without signal peptide, comprising a mutation T713A.
SEQ ID NO: 7 **LFM21GS,** amino acid sequence of a truncated *Clostridium pasteurianum DSM* 525 fructosidase variant without signal peptide, contains the amino acids 454 -937 of the full-length wild-type fructosidase, comprising a mutation T713A.
SEQ ID NO: 8 **LFM29**, Amino acid sequence of a full-length *Lachnospiraceae bacterium* fructosidase LFRU without signal peptide.
SEQ ID NO: 9 **LFM29G,** Amino acid sequence of a full-length *Lachnospiraceae bacterium* fructosidase LFRU without signal peptide, comprising a mutation T607A.
SEQ ID NO: 10 **LFM30,** Amino acid sequence of a full-length *Oscillibacter sp.* fructosidase LFRU without signal peptide.
SEQ ID NO: 11 **LFM30GS,** amino acid sequence of a truncated *Oscillibacter sp.* fructosidase variant without signal peptide, contains the amino acids 1-1350 of the full-length wild-type fructosidase, comprising a mutation T905A.
SEQ ID NO: 12 **LFM37,** Amino acid sequence of a full-length *Ligilactobacillus salivarius* fructosidase LFRU without signal peptide, comprising amino acids 1-973.
SEQ ID NO: 13 **LFM37G,** Amino acid sequence of a full-length *Ligilactobacillus salivarius* fructosidase LFRU without signal peptide, comprising a mutation T666A.
SEQ ID NO: 14 **LFM37S,** amino acid sequence of a truncated *Ligilactobacillus salivarius* fructosidase variant without signal peptide, contains the amino acids 1-855 of the full-length wild-type fructosidase.
SEQ ID NO: 15 **LFM37GS,** amino acid sequence of a truncated *Ligilactobacillus salivarius* fructosidase variant without signal peptide, contains the amino acids 1-855 of the full-length wild-type fructosidase, comprising a mutation T666A.
SEQ ID NO: 16 **LFM39,** Amino acid sequence of a full-length *Ligilactobacillus equi* fructosidase LFRU without signal peptide.
SEQ ID NO: 17 **LFM39GS,** amino acid sequence of a truncated *Ligilactobacillus equi* fructosidase variant without signal peptide, contains the amino acids 1-689 of the full-length wild-type fructosidase, comprising a mutation T503A.
SEQ ID NO: 18 **LFM40,** Amino acid sequence of a full-length *Ligilactobacillus equi* fructosidase LFRU without signal peptide.
SEQ ID NO: 19 **LFM40GS,** amino acid sequence of a truncated *Ligilactobacillus equi* fructosidase variant without signal peptide, contains the amino acids 1-873 of the full-length wild-type fructosidase, comprising a mutation T688A.
SEQ ID NO: 20 amino acid consensus sequence FNGTYNLFL for N-glycosylation.
SEQ ID NO: 21 amino acid consensus sequence FNGTFNLFL for N-glycosylation.
SEQ ID NO: 22 amino acid consensus sequence FNGTYNLNL for N-glycosylation.
SEQ ID NO: 23 amino acid consensus sequence FNGTFNLNL for N-glycosylation.

### DETAILED DESCRIPTION

In the following description, like reference signs denote like elements or steps.

As used herein, the term "fructanase" or "fructosidase" or "exo-beta-D-fructosidase" refers to fructan beta-fructosidase, which is an enzyme with systematic name beta-D-fructan fructohydrolase (EC 3.2.1.80). Fructanase catalyses the hydrolysis of terminal, non-reducing (2->1)- and (2->6)-linked beta-D-fructofuranose residues in fructans. Substrates of fructanase include inulin, levan and sucrose. Fructanase degrades levans and inulins to fructose and also cleaves sucrose into glucose and fructose and can therefore function as an invertase.

As used herein, the term "variant polypeptide" refers to a variant which is a polypeptide. The term "variant polypeptide" used herein may refer to the variant polypeptide of the first aspect, or to another variant polypeptide. As used herein, the term "variant" means a sequence or a fragment of a sequence (nucleotide or amino acid) inserted, substituted, or deleted by one or more nucleotides/amino acids, or which is chemically modified. The term variant may in some embodiments also include the variant polypeptide (of fructanase), and/or the fusion protein including the variant polypeptide (of fructanase).

As used herein, the term "fructanase variant" and "variant fructanase" and "variant of fructanase" refers to a fructanase molecule obtained by site-directed or random mutagenesis, insertion, substitution, deletion, recombination and/or any other suitable protein engineering method, and which leads into a genetically modified fructanase variant that differs in its amino acid sequence from the parent molecule/ fructanase such as a wild type fructanase. The terms "wild type fructanase", "wild type enzyme", "wild type", or "wt" in accordance with the disclosure, describe a fructanase enzyme with an amino acid sequence found in nature or a fragment thereof. The variant encoding gene can be synthesised, or the parent gene be modified using genetic methods, e.g., by site directed mutagenesis, a technique in which one or more than one mutation are introduced at one or more defined sites in a polynucleotide encoding the parent polypeptide.

The term "parent fructanase polypeptide" means a fructanase polypeptide, from which a specific variant fructanase polypeptide is derived from.

The term "variant polypeptide" of fructanase according to the first aspect, may also be referred to by using the name given to variant, e.g., LFM37.

As used herein, a "peptide" and a "polypeptide" are amino acid sequences including a plurality of consecutive polymerized amino acid residues. For purpose of this disclosure, peptides are molecules including up to 20 amino acid residues, and polypeptides include more than 20 amino acid residues. The peptide or polypeptide may include modified amino acid residues, naturally occurring amino acid residues not encoded by a codon, and non-naturally occurring amino acid residues.

As used herein, the term "mature polypeptide" means any polypeptide wherein at least one signal sequence or signal peptide or signal peptide and a putative pro-peptide, or a carrier peptide or a fusion partner is cleaved off. For example, the "mature polypeptide" of variant LFM37 comprises the amino acids 1-973 of SEQ ID NO:12.

The term "secretory signal sequence" or "signal sequence" or a "secretory peptide" refers to an amino acid sequence which is a component or a part of a larger polypeptide, and which directs the larger polypeptide through a secretory pathway of a host cell in which it is produced. The secretory signal sequence can be native, or it can be obtained from another source. Depending on the host cell, the larger polypeptide may be cleaved from the secretory peptide during transit through the secretory pathway, thereby forming a mature polypeptide lacking the secretory peptide.

As used herein, a "protein" may refer to a peptide or a polypeptide of any size. A protein may be an enzyme, a protein, an antibody, a membrane protein, a peptide hormone, regulator, or any other protein.

As used herein, the term "substitution" or "amino acid substitution" means an amino acid residue replacement with an amino acid residue that is different than the original amino acid in that specific replacement position. The term "amino acid substitution" can refer to both, conservative amino acid substitutions and non-conservative amino acid substitutions, which means the amino acid residue is replaced with an amino acid residue having a similar side chain (conservative), or a different side chain (non-conservative), as the original amino acid residue in that place. Substitutions are described using of the following nomenclature: amino acid residue in the protein scaffold; position; substituted amino acid residue(s). According to this nomenclature the substitution of, for instance, a single residue of Threonine to Alanine residue at position 666 is indicated as Thr666Ala or T666A.

By "N-glycosylation" is meant attachment of an oligosaccharide to a nitrogen atom, usually to the nitrogen atom of asparagine residue that is present as a part of Asn-Xaa-Ser/Thr (N-Xaa-S/T) consensus sequence, where Xaa is any amino acid except proline (Pro) present in the polypeptide in question. As used herein, the term "N-glycosylation site" refers to a site in an amino acid chain, wherein the amino acid at that site can be glycosylated.

As used herein, the term "host cell" means any cell type that is susceptible to transformation, transfection, transduction, mating, crossing, CRISPR-Cas or the like with a nucleic acid construct or expression vector comprising a polynucleotide. The term "host cell" encompasses any progeny that is not identical due to mutations that occur during replication. Non-limiting examples of a host cell are cells from the Families *Hypocreacea*, *Nectriaceae*, *Clavicipitaceae, Microascaceae,* preferably from the members of Genera *Aspergillus*, *Chrysosporium*, *Myceliophthora*, *Humicola, Trichoderma* (anamorph of Hypocrea), *Thermothelomyces, Myceliophthora Fusarium, Gibberella, Nectria, Stachybotrys*, *Claviceps, Metarhizium, Villosiclava, Ophiocordyceps, Cephalosporium,* and *Scedosporium;* more preferably from the group consisting of *Trichoderma reesei (Hypocrea jecorina), T. citrinoviridae, T. longibrachiatum, T. virens, T. harzianum, T. asperellum, T. atroviridae, T. parareesei,* , *Fusarium oxysporum, F. gramineanum, F. pseudograminearum, F. venenatum, Gibberella fujikuroi, G. moniliformis,* G. zeaea, *Nectria (Haematonectria) haematococca, Stachybotrys chartarum, S. chlorohalonata, Claviceps purpurea, Metarhizium acridum, M. anisopliae, Villosiclava virens, Ophiocordyceps sinensis, Acremonium (Cephalosporium) chrysogenum, and Scedosporium apiospermum, and Aspergillus niger, Aspergillus awamori, Aspergillus oryzae, Chrysosporium lucknowense, Myceliophthora thermophila, Humicola insolens, Thermothelomyces heterothallica* and *Humicola grisea*, most preferably *Trichoderma reesei.*

Further non-limiting examples of a host cell are bacterial cells, preferably gram-positive Bacilli (e.g., *Bacillus subtilis, Bacillus pumilus, Alkalihalobacillus alcalophilus, Bacillus amyloliquefaciens, Bacillus velezensis, Brevibacillus brevis, Niallia circulans, Shouchella clausii, Weizmannia coagulans, Halalkalibacterium halodurans, Paenibacillus lautus, Lederbergia lenta, Bacillus licheniformis, Bacillus paralicheniformis Priestia megaterium, Geobacillus stearothermophilus, or Bacillus thuringiensis*), gram-negative bacteria (e.g., *Escherichia coli*), actinomycetales (e.g., *Streptomyces* sp., *Nonomuraea flexuosa*) and yeasts (e.g., *Saccharomyces cerevisiae*, *Schizosaccharomyces pombe*, *Pichia pastoris*, *Yarrowia lipolytica).*

As used herein, "sequence identity" means the percentage of exact matches of nucleotides or amino acid residues between two optimally aligned sequences over the number of positions where there are nucleotides/residues present in both sequences. When one sequence has a residue with no corresponding residue in the other sequence, the alignment program allows a gap in the alignment, and that position is not counted in the denominator of the identity calculation. For purposes of the present invention, the sequence identity between two amino acid sequences can be determined using the Needleman-Wunsch algorithm (Needleman et al. 1970) as implemented in the Needle program of the EMBOSS package (Rice et al., 2000), preferably version 5.0.0 or later. The parameters used are gap open penalty of 10, gap extension penalty of 0.5, and the EBLOSUM62 (EMBOSS version of BLOSUM62) substitution matrix. The output of Needle labeled "longest identity" (obtained using the -nobrief option) is used as the percent identity and is calculated as follows: (Identical Residues×100)/(Length of Alignment-Total Number of Gaps in Alignment).

As used herein, "sequence alignment" of the amino acid sequences means, aligning the sequences using Clustal Omega (1.2.4) multiple sequence alignment program (https://www.ebi.ac.uk/Tools/msa/clustalol) as described by Sievers et al. 2011, and using the default settings.

As used herein, the term "corresponding positions" or "corresponding amino acid position" means aligning at least two amino acid sequences according to identified regions of similarity or identity as pairwise alignment or as multiple sequence alignment, thereby pairing up the corresponding amino acids. In the present disclosure corresponding positions typically refers to a position corresponding to the position in SEQ ID NO: 12. The corresponding amino acids between two amino acid sequences need not, but can be, the same amino acids.

Unless otherwise specified, all references to a certain amino acid position refer to an amino acid of the SEQ ID NO: 12 in said position, or to an amino acid present or missing in the corresponding position of an amino acid sequence aligned with SEQ ID NO: 12.

As used herein, the term "fructanase productivity" refers to a level of fructanase production by means of transcription, translation, post-translational modifications and ultimately intracellular or extracellular targeting and/or secretion of the fructanase. Fructanase productivity can thereby refer to the intracellular level of translated fructanase or indicate the level of extracellularly secreted fructanase. Methods for protein production by recombinant technology in different host systems are known in the art. Preferably, the fructanase variants are produced as extracellular proteins that are secreted into the culture medium wherein the host cell is cultured, and from which they can be easily recovered and isolated. Example 3 provides an example of a method for determining fructanase productivity.

As used herein, with "fructanase activity" or "fructan degrading performance" is meant the ability of fructanase to catalyze the hydrolysis of terminal, non-reducing (2->1)- and (2->6)-linked beta-D-fructofuranose residues into fructans. The fructanase activity can be indicated as relative fructanase activity, wherein fructanase activity of a sample is indicated as fold change in relation to the control sample. Example 4 provides an example of a method for determining fructanase activity. The fructanase activity can also be indicated in enzymatic units/ml (U/ml), wherein one enzymatic unit is defined as the amount of the enzyme which produces 1 µmol of reducing sugars per ml.

As used herein, the term "GH32" or the "glycosyl hydrolase 32" refers to a family of enzymes that hydrolyze fructose-containing polysaccharides, comprising at least inulinases, exoinulinases, levanases, 2,6-β-fructan 6-levanbiohydrolases, fructan β-(2,1)-fructosidases and fructan β-(2,6)-fructosidases.

The term "GH32 domain" refers to the catalytic domain of the glycosyl hydrolase 32 enzymes. For estimating the amino acid region comprising the GH32 domain for each fructanase polypeptide, the ColabFold web tool can be used. For example, the ColabFold web tool was used to predict the structure of *Ligilactobacillus salivarius* fructosidase SEQ ID NO: 12, and based on the obtained results the catalytic domain GH32 of the SEQ ID NO: 12 comprises the amino acids 374 - 850.

ColabFold is a web-based implementation of the AlphaFold2 protein folding algorithm, capable of predicting the 3D structure of a protein from its amino acid sequence (Miradita et al., 2022). ColabFold runs on Google Colab, a cloud-based platform for running Jupyter notebooks. Instructions provided in the notebook are used to upload protein sequence in FASTA format. The ColabFold tool performs the following steps: 1) Pre-processing: The input sequence is checked for errors and inconsistencies. 2) Fold recognition: ColabFold first searches a database of known protein structures to identify proteins with similar sequences to input sequence. This information found in the search is used to guide the subsequent structure prediction process. 3) Structure prediction: Using the AlphaFold2 algorithm, ColabFold predicts the 3D structure of protein. The predicted structure is output in PDB format. ColabFold website:
(https://colab.research.google.com/github/sokrypton/ColabFold/blob/main/AlphaFold2.ipyn b).

As used herein, the term "HMM profile" refers to hidden Markov model profiles, which are computational algorithms/models used for predicting protein structure and function, which identify significant protein sequence similarities allowing the detection of homologs. HMM profiles also allow encapsulating the evolutionary changes that have occurred in a set of related sequences (i.e., a multiple sequence alignment). HMM profiles capture position-specific information about how conserved each amino acid is in each column of the alignment. HMM profiles may be generated online, and the HMM profiles may be obtained from the PFAM database. HMM profiles may be generated also locally on a computer using the HMMER software package (current version HMMER 3.3.2 from December 2021). HMMER database is available for all computer platforms from http://hmmer.org/.https://pfam.xfam.org/.

As used herein, the term "C-terminal" or "carboxy-terminal" refers to the end of an amino acid chain (protein, polypeptide, peptide), terminated by a free carboxyl group -COOH. With the term "C-terminal amino acid sequence" is meant an amino acid sequence from which the majority of is located at the C-terminal half of the entire amino acid sequence in question.

As used herein, the term "N-terminal" or "amino-terminal" refers to the end of an amino acid chain (protein, polypeptide or peptide), starting with a free amine group (-NH₂). With the term "N-terminal amino acid sequence" is meant an amino acid sequence from which the majority of is located at the N-terminal half of the entire amino acid sequence in question.

As used herein, the term "plant-based material" refers to materials and products that are at least partially or wholly derived from plants.

With the term "enzyme composition" is meant an enzymatic fermentation product, usually cell-free and possibly isolated and purified, typically produced by a pure culture of a microorganism. The enzyme composition usually comprises a number of different enzymatic activities produced by the microorganism. In another embodiment the enzyme composition is a mixture of monocomponent enzymes, preferably enzymes derived from bacterial or fungal species by using conventional recombinant production techniques. The enzyme composition may contain, for example, stabilators and preservatives which prevent microbial growth and improve storage stability. In an embodiment the term "enzyme composition" means a composition obtained by steps of culturing a host cell and either recovering at least one enzyme, which may be a polypeptide, from the cells or separating the cells from the spent culture medium and obtaining the supernatant comprising the at least one enzyme. The spent culture medium of the production host can be used as such, or the host cells may be removed, and/or it may be concentrated, filtrated or fractionated. It may also be dried. The obtained enzyme composition may be in the form of liquid, powder, granulate or tablets. The enzyme may be in immobilized form in the composition. The enzyme composition may be a monocomponent, or have other enzymes produced by the production host, or a mixture of enzymes from different sources.

The term "carrier" or "carrier polypeptide" refers to a polypeptide into which the protein of interest (fructanase) is translationally fused to improve the yield. The carrier can be either homologous or heterologous to the production host in its origin and can be a full-length protein or a fragment of a protein (e.g., a core, a CBM or a CBM and linker region). It is preferably encoded by a gene or a nucleotide sequence with good expression level.

As used herein, the term "expression" includes any step or all steps involved in the production of a polypeptide in a host cell including, but not limited to, transcription, translation, post-translational modification, and secretion. Expression may be followed by harvesting, i.e., recovering, the host cells or the expressed product.

The following abbreviations are used for amino acids:

| | | |
|---|---|---|
| A | Ala | Alanine |
| C | Cys | Cysteine |
| D | Asp | Aspartic acid |
| E | Glu | Glutamic acid |

| | | |
|---|---|---|
| F | Phe | Phenylalanine |
| G | Gly | Glycine |
| H | His | Histidine |
| I | lie | Isoleucine |
| K | Lys | Lysine |
| L | Leu | Leucine |
| M | Met | Methionine |
| N | Asn | Asparagine |
| P | Pro | Proline |
| Q | Gln | Glutamine |
| R | Arg | Arginine |
| S | Ser | Serine |
| T | Thr | Threonine |
| V | Val | Valine |
| W | Trp | Tryptophan |
| Y | Tyr | Tyrosine |
| X | Xaa | Any amino acid |

As used herein, the term "comprising" includes the broader meanings of "including", "containing", and "comprehending", as well as the narrower expressions "consisting of" and "consisting only of".

The present invention relates to variant polypeptide of a wild type fructanase according to the first aspect.

In an embodiment, the variant polypeptide of a wild type fructanase has: fructanase activity;
an amino acid sequence having at least 65 % amino acid sequence identity with the amino acids 374 - 850 of SEQ ID NO: 12; and at least one substitution, insertion or deletion of an amino acid, the at least one substitution, insertion or deletion preventing N-glycosylation of at least one amino acid when expressed in a host cell capable of N-glycosylation, and/or at least 50 amino acids deleted when compared to the wild type parent fructanase of the variant polypeptide.

In an embodiment, the amino acid numbering of the variant polypeptide corresponds to the amino acid numbering of the SEQ ID NO: 12. The SEQ ID NO:12 is the polypeptide LFM37, indicating a full-length mature *Ligilactobacillus salivarius* fructosidase amino acid sequence without a signal peptide, comprising 973 amino acids. In an embodiment, the variant polypeptide has fructanase activity, preferably the variant polypeptide has an improved fructan degrading performance when compared to its wild type fructanase parent polypeptide.

In an embodiment, the variant polypeptide is an extracellular fructosidase and a variant of glycosyl hydrolase family 32 (GH32) family member, wherein the variant polypeptide has fructanase activity. The catalytic domain GH32 of the SEQ ID NO: 12 comprises the amino acids 374 - 850. In an embodiment, the variant polypeptide has an amino acid sequence having at least 65 % sequence identity with the amino acids comprised by the GH32 domain of the SEQ ID NO: 12. In an embodiment, the variant polypeptide is a GH32 family fructanase which has at least exo activity.

In an embodiment, the amino acid sequence of the variant polypeptide has at least 65 %, but less than 100 % amino acid sequence identity with amino acids 374 - 850 of SEQ ID NO: 12. In an embodiment, the amino acid sequence of the variant polypeptide has 100 % amino acid sequence identity with amino acids 374 - 850 of SEQ ID NO: 12.

In an embodiment, the variant polypeptide is obtained via substitution, insertion or deletion of at least one amino acid of the wild type parent fructanase of the variant polypeptide, the substitution, insertion or deletion of at least one amino acid preventing N-glycosylation of at least one amino acid in the variant polypeptide, when expressed in a host cell capable of N-glycosylation.

In an embodiment, the at least one substitution, insertion or deletion of an amino acid is preventing N-glycosylation of at least one amino acid of the variant polypeptide, as an asparagine (N) present in the N-glycosylation consensus sequence of the parent polypeptide of the variant polypeptide has been deleted or substituted to another amino acid in the variant polypeptide. Thus, in this case the at least one substitution or deletion of an amino acid prevents N-glycosylation of the variant polypeptide by deleting or replacing the asparagine (N) at a specific site (within the N-glycosylation consensus sequence) with another amino acid, thus preventing the N-linked glycosylation of this asparagine (N).

In an embodiment, the variant polypeptide has the at least one substitution, insertion or deletion of an amino acid, the at least one substitution, insertion or deletion preventing N-glycosylation of at least one asparagine (N) of the variant polypeptide, when expressed in a host cell capable of N-glycosylation. Thus, in this case the at least one substitution, insertion or deletion of an amino acid prevents N-glycosylation of the asparagine (N) of the variant polypeptide by destroying the consensus sequence required for N-glycosylation, whereas the variant polypeptide still comprises said asparagine (N).

In an embodiment, the at least one amino acid substitution, insertion or deletion of an amino acid disrupts the consensus sequence for N-glycosylation in the variant polypeptide.

In an embodiment, the variant polypeptide of a wild type fructanase has:
fructanase activity;
an amino acid sequence having at least 65 % amino acid sequence identity with the amino acids 374 - 850 of SEQ ID NO: 12; and
   at least one substitution, insertion or deletion of an amino acid, the at least one substitution, insertion or deletion disrupting at least one N-glycosylation consensus sequence, and/or
   at least 50 amino acids deleted when compared to the wild type parent fructanase of the variant polypeptide.

In an embodiment, the N-glycosylation consensus sequence is Asn-Xaa-Ser/Thr (N-Xaa-S/T), wherein Xaa is any amino acid except proline (Pro). In an embodiment, the N-glycosylation consensus sequence is FNGT(x1)NL, where (x1) is Y or F. In an embodiment, the N-glycosylation consensus sequence is FNGT(x1)NL(x2)L, where (x1) is Y or F and (x2) is For N. In an embodiment, the variant polypeptide does not have an amino acid sequence comprising the N-glycosylation consensus sequence FNGT(x1)NL(x2)L, where (x1) is Y or F and (x2) is F or N.

In an embodiment, the N-glycosylation consensus sequence is FNGTYNLFL or FNGTFNLFL or FNGTYNLNL or FNGTFNLNL. In an embodiment, the variant polypeptide does not have an amino acid sequence comprising the N-glycosylation consensus sequence FNGTYNLFL (SEQ ID NO: 20), or FNGTFNLFL (SEQ ID NO: 21), or FNGTYNLNL (SEQ ID NO: 22), or FNGTFNLNL (SEQ ID NO: 23).

In an embodiment, the amino acid sequence of the variant polypeptide has at least 65 %, but less than 100 % amino acid sequence identity with amino acids 374 - 850 of SEQ ID NO: 12; and at least one substitution, insertion or deletion of an amino acid, the at least one substitution, insertion or deletion preventing N-glycosylation of at least one amino acid of the variant polypeptide, when expressed in a host cell capable of N-glycosylation.

In an embodiment, the amino acid sequence of the variant polypeptide has at least one substitution of an amino acid preventing N-glycosylation of at least one amino acid of the variant polypeptide, when expressed in a host cell capable of N-glycosylation. In an embodiment, the amino acid sequence of the variant polypeptide has at least one insertion of an amino acid preventing N-glycosylation of at least one amino acid of the variant polypeptide, when expressed in a host cell capable of N-glycosylation. In an embodiment, the amino acid sequence of the variant polypeptide has at least one deletion of an amino acid preventing N-glycosylation of at least one amino acid of the variant polypeptide, when expressed in a host cell capable of N-glycosylation. In a preferable embodiment, the at least one substitution, insertion or deletion of an amino acid prevents N-glycosylation of at least asparagine (N) of the variant polypeptide, when expressed in a host cell capable of N-glycosylation.

In an embodiment, the at least one substitution, insertion or deletion of an amino acid prevents the N-glycosylation of at least one amino acid, such as asparagine (N) of the variant polypeptide only when expressed in a host cell capable of N-glycosylation. Cells capable of N-glycosylation generally comprise eukaryotes and archaea, whereas bacterial cells are rarely capable of N-glycosylation, although some bacterial cells can link glycans to amide nitrogens of an asparagines.

In an embodiment, the amino acid sequence of the variant polypeptide has at least 65 % amino acid sequence identity with amino acids 374 - 850 of SEQ ID NO: 12; and at least 50 amino acids deleted when compared to the wild type parent fructanase of the variant polypeptide. In an embodiment, the amino acid sequence of the variant polypeptide has 100 % amino acid sequence identity with amino acids 374 - 850 of SEQ ID NO: 12; and at least 50 amino acids deleted when compared to the wild type parent fructanase of the variant polypeptide. In an embodiment, the amino acid sequence of the variant polypeptide has at least 65 % amino acid sequence identity with amino acids 374 - 850 of SEQ ID NO: 12 and a size of 477 - 2000 amino acids, or 477 - 1418 amino acids, or 477 - 973 amino acids. In an embodiment, the amino acid sequence of the variant polypeptide has at least 65 % amino acid sequence identity with amino acids 374 - 850 of SEQ ID NO: 12; and a deletion of at least 50 amino acids from the amino acid sequence outside the GH32 domain amino acids corresponding to the amino acids 374 - 850 of SEQ ID NO: 12.

In an embodiment, the variant polypeptide of a wild type fructanase has: fructanase activity; an amino acid sequence having at least 65 % amino acid sequence identity with the amino acids 374 - 850 of SEQ ID NO: 12; and at least one substitution of an amino acid preventing N-glycosylation of at least one asparagine (N) of the variant polypeptide when expressed in a host cell capable of N-glycosylation, and/or at least 50 amino acids deleted when compared to the wild type parent fructanase of the variant polypeptide; wherein the amino acid numbering of the variant polypeptide corresponds to the amino acid numbering of the SEQ ID NO: 12.

In an embodiment, the amino acid sequence of the variant polypeptide has the at least one substitution, insertion or deletion of an amino acid, and the at least one amino acid substitution, insertion or deletion is in the amino acid region of the variant polypeptide corresponding to the amino acids 374 - 850 of SEQ ID NO: 12.

In an embodiment, the amino acid sequence of the variant polypeptide has the at least one substitution, insertion or deletion of an amino acid within the amino acid region of the catalytic domain GH32 of the variant polypeptide, the GH32 of the variant polypeptide corresponding to the amino acids 374 - 850 of the SEQ ID NO: 12. In an embodiment, the amino acid sequence of the variant polypeptide has at least one substitution of an amino acid in the amino acid region of the variant polypeptide corresponding to the amino acids 374 - 850 of SEQ ID NO: 12. In an embodiment, the amino acid sequence of the variant polypeptide has at least one deletion of an amino acid in the amino acid region of the variant polypeptide corresponding to the amino acids 374 - 850 of SEQ ID NO: 12. In an embodiment, the amino acid sequence of the variant polypeptide has at least one insertion of an amino acid in the amino acid region of the variant polypeptide corresponding to the amino acids 374 - 850 of SEQ ID NO: 12.

In an embodiment, the amino acid sequence of the variant polypeptide having the at least one substitution, insertion or deletion of an amino acid in the amino acid region of the variant polypeptide corresponding to the amino acids 374 - 850 of SEQ ID NO: 12, preventing N-glycosylation of at least one asparagine (N) of the variant polypeptide when expressed in a host cell capable of N-glycosylation, is beneficial as it has an increased fructanase productivity and/or improved fructan degrading performance, when compared to the parent polypeptide of the variant polypeptide, or to the polypeptide having the sequence SEQ ID NO: 12, preferably the variant polypeptide has at least an improved fructan degrading performance.

In a preferable embodiment, the at least one amino acid substitution, insertion or deletion comprised by the amino acid sequence of the variant polypeptide disrupts a consensus sequence N-X-S/T for N-glycosylation, wherein X is any amino acid except proline (Pro), and S/T means the amino acid may be either Serine or Threonine. In an embodiment, the variant polypeptide comprises the at least one substitution, insertion or deletion within the consensus sequence N-X-S/T for N-glycosylation, the at least one substitution, insertion or deletion preventing N-glycosylation of asparagine (N) of said consensus sequence. In an embodiment, the at least one amino acid substitution, insertion or deletion comprised by the amino acid sequence of the variant polypeptide disrupts a consensus sequence N-X-S/T for N-glycosylation at the amino acid positions corresponding to the amino acid positions 664 - 666 of the SEQ ID NO: 12. In an embodiment, the variant polypeptide comprises the at least one substitution, insertion or deletion within the consensus sequence N-X-S/T for N-glycosylation, the at least one substitution, insertion or deletion preventing N-glycosylation of asparagine (N) at the amino acid position corresponding to the amino acid position 664 of the SEQ ID NO: 12. In an embodiment, the amino acid sequence of the variant polypeptide does not comprise the consensus sequence for N-glycosylation N-X-S/T at the amino acid positions corresponding to the amino acid positions 664 - 666 of the SEQ ID NO: 12. In an embodiment, the amino acid sequence of the variant polypeptide comprises a disrupted consensus sequence for N-glycosylation at the amino acid positions corresponding to the amino acid positions 664 - 666 of the SEQ ID NO: 12.

In an embodiment, the amino acid sequence of the variant polypeptide has the at least one substitution, insertion or deletion of an amino acid, and wherein the at least one amino acid substitution, insertion or deletion disrupts a consensus sequence for N-glycosylation FNGT(x1)NL(x2)L, where (x1) is Y or F, and (x2) is N or F.

In an embodiment, the at least one amino acid substitution, insertion or deletion comprised by the amino acid sequence of the variant polypeptide disrupts a consensus sequence for N-glycosylation FNGT(x1)NL(x2)L, where (x1) is Y or F, and (x2) is N or F, at the amino acid positions corresponding to the amino acid positions 663 - 671 of the SEQ ID NO: 12. In an embodiment, the amino acid sequence of the variant polypeptide comprises a disrupted consensus sequence for N-glycosylation at the amino acid positions corresponding to the amino acid positions 663 - 671 of the SEQ ID NO: 12.

In an embodiment, the amino acid sequence of the variant polypeptide has the at least one substitution, insertion or deletion of an amino acid, comprising at least one substitution or deletion of an amino acid comprised by the consensus sequence for N-glycosylation FNGT(x1)NL(x2)L, where (x1) is Y or F, and (x2) is N or F. In an embodiment, the amino acid sequence of the variant polypeptide has the at least one substitution, insertion or deletion of an amino acid, comprising at least one amino acid insertion to the consensus sequence for N-glycosylation FNGT(x1)NL(x2)L, where (x1) is Y or F, and (x2) is N or F.

In an embodiment, the amino acid sequence of the variant polypeptide has the at least one substitution, insertion or deletion of an amino acid, wherein the at least one amino acid substitution, insertion or deletion comprises a substitution or deletion of Threonine (T) in a consensus sequence for N-glycosylation FNGT(x1)NL(x2)L, where (x1) is Y or F, and (x2) is N or F.

In an embodiment, the amino acid sequence of the variant polypeptide has the at least one substitution, insertion or deletion of an amino acid, wherein the at least one amino acid substitution, insertion or deletion comprises a substitution or deletion of Threonine (T) in a consensus sequence for N-glycosylation of FNGTYNLNL, FNGTFNLNL, FNGTYNLFL, or FNGTFNLFL.

In an embodiment, wherein the at least one amino acid substitution, insertion or deletion comprises a substitution or deletion of Threonine (T) in a consensus sequence for N-glycosylation FNGT(x1)NL(x2)L, where (x1) is Y or F, and (x2) is N or F, said substitution or deletion of Threonine (T) prevents N-glycosylation of at least one asparagine (N) of the variant polypeptide, when expressed in a host cells capable of N-glycosylation.

In an embodiment, the amino acid sequence of the variant polypeptide has the at least one substitution, insertion or deletion of an amino acid, which comprises a substitution or deletion of Threonine (T) at amino acid position corresponding to amino acid position 666 of the SEQ ID NO: 12, wherein the T is part of the consensus sequence for N-glycosylation FNGT(x1)NL(x2)L, where (x1) is Y or F, and (x2) is N or F. In an embodiment, the amino acid sequence of the variant polypeptide has the at least one substitution, insertion or deletion of an amino acid, which comprises a substitution of Threonine (T) to any other amino acid except S or T at amino acid position corresponding to amino acid position 666 of the SEQ ID NO: 12, wherein the T is part of the consensus sequence for N-glycosylation FNGT(x1)NL(x2)L, where (x1) is Y or F, and (x2) is N or F.

In an embodiment, the amino acid sequence of the variant polypeptide has the at least one substitution, insertion or deletion of an amino acid, which comprises a substitution or deletion of Asparagine (N) in the consensus sequence for N-glycosylation FNGT(x1)NL(x2)L, where (x1) is Y or F, and (x2) is N or F. In an embodiment, the amino acid sequence of the variant polypeptide has the at least one substitution, insertion or deletion of an amino acid, which comprises a substitution or a deletion of Asparagine (N) at amino acid position corresponding to amino acid position 664 of the SEQ ID NO: 12, wherein the N is part of the consensus sequence for N-glycosylation FNGT(x1)NL(x2)L, where (x1) is Y or F, and (x2) is N or F.

In an embodiment, the amino acid sequence of the variant polypeptide has the at least one substitution, insertion or deletion of an amino acid, comprising at least a substitution or deletion of Glycine (G) in the consensus sequence for N-glycosylation FNGT(x1)NL(x2)L, where (x1) is Y or F, and (x2) is N or F. In an embodiment, the amino acid sequence of the variant polypeptide has the at least one substitution, insertion or deletion of an amino acid, comprising at least a substitution of Glycine (G) to Proline (P), at amino acid position corresponding to amino acid position 665 of the SEQ ID NO: 12, wherein the G is part of the consensus sequence for N-glycosylation FNGT(x1)NL(x2)L, where (x1) is Y or F, and (x2) is N or F.

In an embodiment, the amino acid sequence of the variant polypeptide has the at least one substitution, insertion, or deletion of an amino acid, which comprises an insertion of at least one amino acid between amino acids N and G, or between amino acids G and T, or combination thereof, in the consensus sequence for N-glycosylation FNGT(x1)NL(x2)L, where (x1) is Y or F, and (x2) is N or F. In an embodiment, the amino acid sequence of the variant polypeptide has the at least one substitution, insertion or deletion of an amino acid, which comprises an insertion of at least one amino acid between amino acids N and G, in the consensus sequence for N-glycosylation FNGT(x1)NL(x2)L, where (x1) is Y or F, and (x2) is N or F, and wherein the at least one inserted amino acid can be any amino acid. In an embodiment, the amino acid sequence of the variant polypeptide has the at least one substitution, insertion or deletion of an amino acid, which comprises an insertion of at least one amino acid between amino acids corresponding to the amino acids N664 and G665 of the SEQ ID NO: 12, in the consensus sequence for N-glycosylation FNGT(x1)NL(x2)L, where (x1) is Y or F, and (x2) is N or F, wherein the at least one inserted amino acid can be any amino acid.

In an embodiment, the amino acid sequence of the variant polypeptide has the at least one substitution, insertion or deletion of an amino acid, which comprises an insertion of at least one amino acid between amino acids G and T, in the consensus sequence for N-glycosylation FNGT(x1)NL(x2)L, where (x1) is Y or F, and (x2) is N or F, and wherein the at least one inserted amino acid can be any amino acid except S or T. In an embodiment, the amino acid sequence of the variant polypeptide has the at least one substitution, insertion or deletion of an amino acid, which comprises an insertion of at least one amino acid between amino acids corresponding to the amino acids G665 and T666 of the SEQ ID NO: 12, in the consensus sequence for N-glycosylation FNGT(x1)NL(x2)L, where (x1) is Y or F, and (x2) is N or F, wherein the at least one inserted amino acid can be any amino acid except S or T.

In an embodiment, the amino acid sequence of the variant polypeptide has the at least one substitution, insertion or deletion of an amino acid, which comprises at least one amino acid insertion between amino acid positions corresponding to the amino acid positions 664 and 665, or between amino acid positions corresponding to the amino acid positions 665 and 666 of the SEQ ID NO: 12, or combination thereof.

It is understood, that disrupting the consensus sequence for N-glycosylation of the variant polypeptide can be obtained in alternative ways, e.g. by substitution, insertion or deletion of at least one amino acid of the consensus sequence for N-glycosylation, comprised by the variant polypeptide. Regardless of the exact mechanism how the disruption of said consensus sequence for N-glycosylation takes place, the N-glycosylation within this consensus sequence of the variant polypeptide is prevented.

The disruption of the N-glycosylation consensus sequence FNGT(x1)NL(x2)L, where (x1) is Y or F and (x2) is N or F, in the variant polypeptide is beneficial at least because it increases the fructanase productivity and/or improves fructan degrading performance of the variant polypeptide when compared to a polypeptide having the sequence SEQ ID NO: 12, or when compared to the wild type parent fructanase of the variant polypeptide.

In an embodiment, the amino acid sequence of the variant polypeptide has the at least one substitution, insertion or deletion of an amino acid, the at least one substitution, insertion or deletion of an amino acid comprising at least one amino acid substitution or deletion at amino acid position corresponding to any of the amino acid positions 664 - 666 of the SEQ ID NO: 12.

In an embodiment, the amino acid sequence of the variant polypeptide has the at least one substitution, insertion or deletion of an amino acid, which comprises at least one amino acid substitution of the variant polypeptide at amino acid position corresponding to the amino acid position 664, 665, or 666 of the SEQ ID NO: 12, or any combination thereof. In an embodiment, the amino acid sequence of the variant polypeptide has the at least one substitution, insertion or deletion of an amino acid, which is at least one amino acid deletion of the variant polypeptide at amino acid position corresponding to the amino acid position 664, 665, or 666 of the SEQ ID NO: 12, or any combination thereof.

In an embodiment, the amino acid sequence of the variant comprises at least one amino acid substitution at amino acid position corresponding to the amino acid position 666 of the SEQ ID NO: 12. In an embodiment, the amino acid sequence of the variant polypeptide comprises at least one amino acid deletion at amino acid position corresponding to the amino acid position 666 of the SEQ ID NO: 12. In a preferable embodiment, the amino acid sequence of the variant polypeptide comprises an amino acid substitution of Threonine (T) to Alanine (A), at amino acid position corresponding to the amino acid position 666 of the SEQ ID NO: 12. In an embodiment, the amino acid sequence of the variant polypeptide comprises an amino acid deletion of Threonine (T), at amino acid position corresponding to the amino acid position 666 of the SEQ ID NO: 12.

In an embodiment, the amino acid sequence of the variant polypeptide has at least one substitution, insertion or deletion of an amino acid preventing N-glycosylation of the asparagine (N) at the amino acid position corresponding to the amino acid position 664 of the SEQ ID NO: 12. In an embodiment, the amino acid sequence of the variant polypeptide has at least one substitution of an amino acid preventing N-glycosylation of the amino acid at the amino acid position corresponding to the amino acid position 664 of the SEQ ID NO: 12. In an embodiment, the amino acid sequence of the variant polypeptide has at least one insertion of an amino acid preventing N-glycosylation of the amino acid at the amino acid position corresponding to the amino acid position 664 of the SEQ ID NO: 12. In an embodiment, the amino acid sequence of the variant polypeptide has at least one deletion of an amino acid preventing N-glycosylation of the amino acid at the amino acid position corresponding to the amino acid position 664 of the SEQ ID NO: 12. In an embodiment, the variant polypeptide comprises at least one further substitution, insertion or deletion of an amino acid preventing N-glycosylation at a different amino acid position than at the position corresponding to the amino acid position 664 of the SEQ ID NO: 12, when expressed in a host cell capable of N-glycosylation. In an embodiment, the variant polypeptide comprises plurality of amino acid substitutions, insertions and/or deletions, which prevent N-glycosylation of plurality of amino acids comprised by the variant polypeptide, when expressed in a host cell capable of N-glycosylation.

In an embodiment, the variant polypeptide having the at least one amino acid substitution or deletion at amino acid position corresponding to the amino acid position 666 of the SEQ ID NO: 12 is beneficial, as the variant polypeptide comprises a disrupted N-glycosylation consensus sequence. It is understood, that disrupting the consensus sequence for N-glycosylation of the variant polypeptide can be obtained in alternative ways, e.g. by substituting or deleting at least one amino acid at a position corresponding to any of the amino acid positions 664 - 666 of the SEQ ID NO: 12, or by inserting at least one amino acid between amino acids corresponding to the amino acids 664 and 665, or between amino acids corresponding to the amino acids 665 and 666 of the SEQ ID NO: 12. Regardless of the exact mechanism how the disruption of said consensus sequence for N-glycosylation takes place, the N-glycosylation within the consensus sequence of the variant polypeptide is prevented.

In an embodiment, the amino acid sequence of the variant polypeptide comprises at least the amino acids corresponding to the amino acids 374 - 850 of the SEQ ID NO: 12.

In an embodiment, the amino acid sequence of the variant polypeptide comprises the at least one substitution or insertion of an amino acid, and at least the amino acids corresponding to the amino acids 374 - 850 of the SEQ ID NO: 12. In an alternative embodiment, the amino acid sequence of the variant polypeptide comprises the at least one deletion of an amino acid, wherein the at least one amino acid is deleted from the amino acid region corresponding to the amino acids 374 - 850 of the SEQ ID NO: 12.

In an embodiment, the amino acid sequence of the variant polypeptide does not have the at least one substitution, insertion or deletion of an amino acid, and comprises at least the amino acids corresponding to the amino acids 374 - 850 of the SEQ ID NO: 12. In such an embodiment, the variant polypeptide has the at least 50 amino acids deleted from its amino acid sequence when compared to the wild type parent fructanase of said variant polypeptide.

In an embodiment, the amino acid sequence of the variant polypeptide has at least 50 amino acids deleted, and the amino acid sequence has the at least 50 amino acids deleted, or at least 100 amino acids deleted, from the C-terminal side of an amino acid corresponding to the amino acid 850 of the SEQ ID NO: 12 and/or from the N-terminal side of an amino acid corresponding to the amino acid 374 of the SEQ ID NO: 12, preferably from the C-terminal side of an amino acid corresponding to the amino acid 850 of the SEQ ID NO: 12. In an embodiment, the variant polypeptide has the at least 50 amino acids deleted from its N-terminal amino acid sequence or end. In an embodiment, the variant polypeptide has, in addition to a C-terminal deletion of amino acids, also amino acids deleted from its N-terminal amino acid sequence or end.

In an embodiment, the amino acid sequence of the variant polypeptide comprising at least the amino acids corresponding to the amino acids 374 - 850 of the SEQ ID NO: 12 is beneficial as the variant polypeptide has fructanase activity, preferably increased fructanase activity when compared to the wild type parent fructanase polypeptide of the variant.

In an embodiment, the variant polypeptide has the at least 50 amino acids deleted from its amino acid sequence, wherein the at least 50 amino acids, or at least 100 amino acids are deleted in amino acid positions corresponding to the amino acid positions 1 - 373, and/or positions 851 - 973 of SEQ ID NO: 12, preferably corresponding to the amino acid positions 851 - 973 of SEQ ID NO: 12.

In a preferable embodiment, the amino acid sequence of the variant polypeptide has at least 50 amino acids deleted in amino acid positions corresponding to the amino acid positions 851 - 973 of SEQ ID NO: 12.

In an embodiment, the amino acid sequence of the variant polypeptide has at least 100 amino acids deleted in amino acid positions corresponding to the amino acid positions 1 - 373 and/or amino acid positions 851 - 973 of SEQ ID NO: 12. In a preferable embodiment, the amino acid sequence of the variant polypeptide has at least 100 amino acids deleted in amino acid positions corresponding to the amino acid positions 851 - 973 of SEQ ID NO: 12.

In an embodiment, the amino acid sequence of the variant polypeptide has at least 50 amino acids deleted in amino acid positions corresponding to the amino acid positions 1 - 373 and/or amino acid positions 851 - 973 of SEQ ID NO: 12, wherein the at least 50 deleted amino acids is a sum of two or more deleted separate parts of the amino acid sequence. By separate parts of the amino acid sequence in this respect is meant stretches of the amino acid sequence, separated from each other by amino acids which are not deleted. This means, amino acids are deleted from two or more amino acid regions of the wild type parent polypeptide. For example, the variant LFM38GS (SEQ ID NO: 4) has the N-terminal amino acids 1-150 and the C-terminal amino acids 1182 -1418 deleted, which deleted amino acids are present in the parent polypeptide LFM38.

In an embodiment, the variant polypeptide has at least 50 amino acids less than its wild type parent polypeptide, wherein the amino acids of the variant polypeptide are deleted from the amino acid positions which at least partially correspond to the amino acid positions 1 - 373, and/or positions 851 - 973 of SEQ ID NO: 12. In an alternative embodiment, the variant polypeptide has at least 50 amino acids less than its wild type parent polypeptide, wherein at least some of the amino acids of the variant polypeptide are deleted from positions which do not correspond with any of the amino acids of SEQ ID NO: 12.

In an embodiment, the variant polypeptide has the amino acids which correspond to the amino acids 851 - 973 of SEQ ID NO: 12 deleted from its amino acid sequence. In an embodiment, the variant polypeptide has the amino acids which correspond to the amino acids 1 - 373 of SEQ ID NO: 12 deleted from its amino acid sequence.

In an embodiment, the amino acid sequence of the variant polypeptide has 100 - 1000, preferably 100 - 800, more preferably 100 - 500, even more preferably 100 - 400 amino acids deleted from the amino acid sequence of its wild type parent polypeptide, in amino acid positions at least partly corresponding to the amino acid positions 1 - 373 and/or amino acid positions 851 - 973 of SEQ ID NO: 12. In an embodiment, the amino acid sequence of the variant polypeptide has 100 - 1000, preferably 100 - 800, more preferably 100 - 500, even more preferably 100 - 400 amino acids deleted from the amino acid sequence of its wild type parent polypeptide, in amino acid positions at least partly corresponding to the amino acid positions 851 - 973 of SEQ ID NO: 12. In an embodiment, the amino acid sequence of the variant polypeptide has more than 100 amino acids, such as 118, 121, 138, 176, 388 or 397 amino acids deleted from the amino acid sequence of its wild type parent polypeptide, in amino acid positions at least partly corresponding to the amino acid positions 1 - 373 and/or amino acid positions 851 - 973 of SEQ ID NO: 12.

In an embodiment, the variant polypeptide has the at least 50 amino acids deleted from its amino acid sequence at its C-terminal end, or at its N-terminal end, or combinations thereof. In an embodiment, the fructanase productivity and/or the relative fructanase activity of the variant polypeptide is increased, when the variant polypeptide has the at least 50 amino acids deleted from its amino acid sequence at its C-terminal end, or at its N-terminal end, as long as the at least 50 amino acids are not deleted from the GH32 domain of the variant. Thus, the fructanase productivity and/or the relative fructanase activity are increased, when the variant polypeptide has at least 50 amino acids deleted from its terminal regions, which terminal regions do not overlap with the GH32 domain of the variant polypeptide.

In an embodiment, the fructanase productivity and/or relative fructanase activity of the variant polypeptide having at least 50 amino acids deleted from its C-terminal end and/or from its N-terminal end, is increased when compared to its wild type parent polypeptide and/or to fructanase polypeptide of SEQ ID NO: 12.

In an embodiment, the amino acid sequence of the variant polypeptide has at least 70 %, preferably at least 75 %, more preferably at least 80 %, even more preferably at least 85 % amino acid sequence identity with the amino acids 374 - 850 of SEQ ID NO: 12.

In an embodiment, the amino acid sequence of the variant polypeptide has at least 88 %, preferably at least 90 %, more preferably at least 95 %, even more preferably at least 98 %, most preferably at least 99 % amino acid sequence identity with the amino acids 374 - 850 of SEQ ID NO: 12.

In an embodiment, the variant polypeptide has an amino acid sequence having at least 65 %, sequence identity with the amino acids comprised by the GH32 domain of the SEQ ID NO: 12. In an embodiment, the variant polypeptide has an amino acid sequence having at least 70 %, preferably at least 75 %, more preferably at least 80 %, even more preferably at least 85 % sequence identity with the amino acids comprised by the GH32 domain of the SEQ ID NO: 12.

In an embodiment, the amino acid sequence of the variant polypeptide has at least 70 %, at least 75 %, at least 80 %, or at least 85 % amino acid sequence identity with the amino acids 374 - 850 of SEQ ID NO: 12. In an embodiment, the amino acid sequence of the variant polypeptide has 65 % - 100%, 70 % - 100 %, 75 % - 100 %, 80 % - 100 %, or 85 % - 100 % amino acid sequence identity with the amino acids 374 - 850 of SEQ ID NO: 12. In an embodiment, the amino acid sequence of the variant polypeptide has at least 66 %, at least 67 %, at least 69 %, at least 71 %, at least 72 %, or at least 76 % amino acid sequence identity with the amino acids 374 - 850 of SEQ ID NO: 12. In an embodiment, the amino acid sequence of the variant polypeptide has 65 % - 100%, 66 % -100 %, 67 % - 100 %, 69 % - 100 %, 71 % - 100 %, 72 % - 100 %, or 76 % - 100 % amino acid sequence identity with the amino acids 374 - 850 of SEQ ID NO: 12.

In a preferable embodiment, the amino acid sequence of the variant polypeptide has at least 65 %, but less than 100 % amino acid sequence identity with the amino acids 374 - 850 of SEQ ID NO: 12.

In an embodiment, the amino acid sequence of the variant polypeptide has at least 65 %, at least 70 %, at least 75 %, at least 80 %, at least 85 %, at least 88 %, at least 90 %, at least 95 %, at least 98 %, at least 99 %, or 100 % amino acid sequence identity with the amino acids 1 - 973 of SEQ ID NO: 12. In an embodiment, the amino acid sequence of the variant polypeptide has at least 50 amino acids deleted when compared to the wild type parent fructanase of the variant polypeptide, and the amino acid sequence of the variant polypeptide has 100 % amino acid sequence identity with the amino acids 1 - 973 of SEQ ID NO: 12.

In an embodiment, the amino acid sequence of the variant polypeptide has at least 65 % but less than 100 % amino acid sequence identity with the amino acids of its wild type parent polypeptide, preferably the amino acid sequence of the variant polypeptide has high, such as at least 95 %, at least 98 % or at least 99% amino acid sequence identity with the amino acids of its wild type parent polypeptide. In an embodiment, the amino acid sequence of the variant polypeptide has 100 % amino acid sequence identity with the amino acids of its wild type parent polypeptide.

In an embodiment, the minimum size of the variant polypeptide is determined by the requirements of the variant polypeptide having fructanase activity and an amino acid sequence having at least 65 % amino acid sequence identity with amino acids 374 - 850 of SEQ ID NO: 12. In an exemplary embodiment, the amino acid sequence of the variant polypeptide has at least 65 % amino acid sequence identity with amino acids 374 - 850 of SEQ ID NO: 12 and a size of 477 - 1471 amino acids.

In an embodiment, the variant polypeptide has a substrate specificity to inulin over levan. In an embodiment, the variant polypeptide has a substrate specificity ratio of inulin:levan of >1. In an embodiment, the variant polypeptide has a substrate specificity ratio of inulin:levan of 1.2 or higher, preferably 1.5 or higher, more preferably 1.8 or higher, even more preferably 2 or higher, most preferably 2.4 or higher. In an alternative embodiment, the variant polypeptide has a substrate specificity to levan over inulin.

In an embodiment, the variant polypeptide is of bacterial origin, preferably originating from the family *Lactobacillaceae*, *Oscillospiraceae*, *Lachnospiraceae, Clostridiaceae*, *Streptococcacea*, *Atopobiaceae, Bacillaceae or Neocallimastigaceae* wild type bacterial fructanases, more preferably originating from the genus *Ligilactobacillus, Oscillibacter, Clostridium, Streptococcus, Lancefieldella, Lactobacillus*, *Bacillus, Alteribacillus* or *Anaeromyces* wild type bacterial fructanases, most preferably from the species *Ligilactobacillus salivarius* wild type fructanase. In an embodiment, the variant polypeptide is of bacterial origin, meaning the wild type fructanase parent polypeptide of the variant polypeptide is a bacterial fructanase. In an embodiment, the variant polypeptide is of bacterial origin, preferably originating from the family *Lactobacillaceae*, *Oscillospiraceae*, *Lachnospiraceae, Clostridiaceae*, or *Streptococcacea* wild type bacterial fructanases. In an embodiment, the variant polypeptide originates from the genus *Ligilactobacillus*, Oscillibacter, Clostridium, or Streptococcus wild type bacterial fructanases, most preferably from the species Ligilactobacillus salivarius wild type fructanase. In an embodiment, the variant polypeptide originates from a wild type bacterial fructanase of the species *Ligilactobacillus salivarius.*

Therefore, the variant polypeptide of a wild type fructanase is not bound or restricted to a specific family, genus, or species of origin, but can have also different origin than the wild type fructanase of SEQ ID NO: 12, for example. Instead of the family, genus, or species of origin, the variant polypeptide is defined through having fructanase activity, an amino acid sequence having at least 65 % amino acid sequence identity with the GH32 domain of SEQ ID NO: 12; and at least one substitution, insertion or deletion of an amino acid and/or (instead of, or in addition to the at least one substitution, insertion or deletion of an amino acid the variant polypeptide) at least 50 amino acids deleted when compared to the wild type parent fructanase of the variant polypeptide.

In an embodiment, the variant polypeptide has an increased fructanase productivity and/or improved fructan degrading performance when compared to a polypeptide having the sequence SEQ ID NO: 12, or when compared to a wild type parent fructanase of the variant polypeptide. For purposes of the present invention, fructanase productivity may be determined according to the method described in Example 3 below, and the fructan degrading performance (activity) may be determined according to the method described in Example 4 below.

In an embodiment, the variant polypeptide has an increased fructanase productivity when compared to a polypeptide having the sequence SEQ ID NO: 12, or when compared to a wild type parent fructanase of the variant polypeptide.

In an embodiment, the relative fructanase productivity of the variant polypeptide is at least 1.5, preferably at least 2, more preferably at least 3, more preferably at least 4, even more preferably at least 6 fold higher when compared to a polypeptide having the sequence SEQ ID NO: 12, or when compared to the wild type parent fructanase of the variant polypeptide. In a preferable embodiment, the relative fructanase productivity of the variant polypeptide is at least 10, preferably at least 20, more preferably at least 30 fold higher when compared to a polypeptide having the sequence SEQ ID NO: 12, or when compared to the wild type parent fructanase of the variant polypeptide. By fructanase productivity fold change, such as increase, in this respect is meant the ratio between the fructanase productivity of the measured variant (B) and fructanase productivity of the comparison sample (A), e.g. the polypeptide having the sequence SEQ ID NO: 12. Thus for fructanase productivities of (A) and (B) the fold change of B with respect to A is B/A.

In an embodiment, the fructanase productivity of the variant polypeptide is higher when compared to a polypeptide having the sequence SEQ ID NO: 12, or when compared to the wild type parent fructanase of the variant polypeptide, wherein the higher fructanase productivity of the variant polypeptide is independent of the host cell wherein the variant polypeptide is produced.

In an embodiment, the variant polypeptide has an improved fructan degrading performance, when compared to a polypeptide having the sequence SEQ ID NO: 12 or when compared to the wild type parent fructanase of the variant polypeptide. With the improved fructan degrading performance is meant, the variant polypeptide has a higher fructanase activity when compared to a polypeptide having the sequence SEQ ID NO: 12 or when compared to the wild type parent fructanase of the variant polypeptide.

In an embodiment, the relative fructanase activity of the variant polypeptide is at least 1, preferably at least 2, more preferably at least 3, more preferably at least 4, more preferably at least 5, more preferably at least 6, even more preferably at least 7 fold higher, when compared to a polypeptide having the sequence SEQ ID NO: 12, or when compared to the wild type parent fructanase of the variant polypeptide. In an embodiment, the fructanase activity is referred to as relative fructanase activity. In an embodiment, the fructanase activity of the variant polypeptide is at least 10, preferably at least 20, more preferably at least 50, even more preferably at least 100 fold higher when compared to a polypeptide having the sequence SEQ ID NO: 12 or when compared to the wild type parent fructanase of the variant polypeptide. In an exemplary embodiment, the variant polypeptide has at least 2.9 fold, at least 43 fold, at least 93 fold, at least 108 fold, or at least 449 fold higher fructanase activity when compared to its wild type parent fructanase of the variant polypeptide.

By fructanase activity fold change, such as increase, in this respect is meant the ratio between the fructanase activity of the measured variant (B) and fructanase activity of the comparison sample (A), such as the polypeptide having the sequence SEQ ID NO: 12. Thus for fructanase activities of (A) and (B) the fold change of B with respect to A is B/A.

In an embodiment, the increased fructanase productivity of the variant polypeptide means, the variant polypeptide also has an improved fructan degrading performance, when compared to a polypeptide having the sequence SEQ ID NO: 12 or when compared to a wild type parent fructanase of the variant polypeptide, as the quantity of the variant polypeptide is increased.

In an embodiment, the variant polypeptide has an improved fructan degrading performance when compared to a polypeptide having the sequence SEQ ID NO: 12 or when compared to the wild type parent fructanase of the variant polypeptide, wherein the improved fructan degrading performance of the variant polypeptide is independent of the host cell wherein the variant polypeptide is produced. In an embodiment, the variant polypeptide has an improved fructan degrading performance when compared to a polypeptide having the sequence SEQ ID NO: 12 or when compared to the wild type parent fructanase of the variant polypeptide, when produced in a fungal or a bacterial cell.

In an embodiment, the variant polypeptide having the at least one substitution, insertion or deletion of an amino acid preventing N-glycosylation of at least one amino acid of the variant polypeptide, is beneficial, as the variant polypeptide has a higher fructanase productivity and/or activity, when compared to a polypeptide having the sequence SEQ ID NO: 12, or when compared to the wild type parent fructanase of the variant polypeptide. In an embodiment, the variant polypeptide having the at least one substitution, insertion or deletion of an amino acid preventing N-glycosylation of at least one amino acid of the variant polypeptide has a higher fructanase productivity and/or activity, when compared to its wild type fructanase parent polypeptide having the at least one amino acid N-glycosylated. In an embodiment, the variant polypeptide having the at least one substitution, insertion or deletion of an amino acid preventing N-glycosylation of at least one amino acid within amino acid region corresponding to amino acids 374 - 850 of SEQ ID NO: 12, has a higher fructanase productivity and/or activity, when compared to a corresponding variant polypeptide having the at least one amino acid N-glycosylated. In an embodiment, the variant polypeptide having the at least one substitution, insertion or deletion of an amino acid preventing N-glycosylation of at least one asparagine (N) within the consensus sequence for N-glycosylation FNGT(x1)NL(x2)L, where (x1) is Y or F, and (x2) is N or F, has a higher fructanase productivity and/or activity, when compared to a corresponding variant polypeptide having the at least one asparagine (N) N-glycosylated. In an embodiment, prevention of the N-glycosylation of at least one asparagine (N) within the consensus sequence for N-glycosylation FNGT(x1)NL(x2)L in the variant polypeptide, takes place either by deletion or substitution of said asparagine (N), or by substitution, insertion or deletion of another amino acid within said consensus sequence for N-glycosylation, thereby preventing N-glycosylation of the asparagine (N). In an embodiment, the variant polypeptide having the at least one substitution, insertion or deletion of an amino acid preventing N-glycosylation of at least one asparagine (N), and having at least one amino acid substitution or deletion at amino acid position corresponding to the amino acid position 666 of the SEQ ID NO: 12, has a higher fructanase productivity and/or activity, when compared to a corresponding polypeptide having the at least one asparagine (N) N-glycosylated.

In an embodiment, the variant polypeptide having the at least 50 amino acids deleted when compared to the wild type parent fructanase of the variant polypeptide is beneficial, as the variant polypeptide has a higher fructanase productivity and/or activity, when compared to a polypeptide having the sequence SEQ ID NO: 12, or when compared to the wild type parent fructanase of the variant polypeptide. In an embodiment, the variant polypeptide having the at least 50 amino acids deleted in amino acid positions corresponding to the amino acid positions 1 - 373, and/or positions 851 - 973 of SEQ ID NO: 12, has a higher fructanase productivity and/or activity, when compared to a polypeptide having the sequence SEQ ID NO: 12, or when compared to the wild type parent fructanase of the variant polypeptide. In an embodiment, the variant polypeptide having an amino acid sequence having at least 65 % amino acid sequence identity with the amino acids 374 - 850 of SEQ ID NO: 12 and the at least 50 amino acids deleted in amino acid positions corresponding to the amino acid positions 851 - 973 of SEQ ID NO: 12, has a higher fructanase productivity and/or activity, when compared to a polypeptide having the sequence SEQ ID NO: 12, or when compared to the wild type parent fructanase of the variant polypeptide.

Therefore, both, the at least one substitution, insertion, or deletion of an amino acid preventing N-glycosylation of at least one amino acid, and the deletion of at least 50 amino acids, independently increase the fructanase productivity and/or activity of the variant polypeptide. In an embodiment, the variant polypeptide having the at least one substitution, insertion or deletion of an amino acid preventing N-glycosylation of at least one amino acid; and being obtained via deletion of at least 50 amino acids from its wild type parent fructanase, has at least increased fructanase productivity or an increased fructanase activity, when compared to the wild type parent fructanase of the variant polypeptide, preferably at least increased fructanase productivity. Therefore, a variant polypeptide comprising both, the at least one substitution, insertion, or deletion of an amino acid preventing N-glycosylation of at least one amino acid, and the deletion of at least 50 amino acids, is beneficial as it has even more increased fructanase productivity and/or activity of the variant polypeptide, when compared to variant polypeptides having only one of these modifications. This can be observed, for example, from the Tables 2 and 3 with the molecules LFM37, LFM37G, LFM37S and LFM37GS.

In an embodiment, the relative fructanase activity and/or productivity of the variant polypeptide is independent of the species of origin of the variant polypeptide.

In an embodiment, a fusion protein comprises the variant polypeptide and at least one: an amino acid sequence providing a secretory signal sequence; an amino acid sequence which facilitates purification, such as an affinity tag or His-tag; an amino acid sequence controlling and enhancing the production of the fusion protein, such as a carrier polypeptide; an amino acid sequence encoding a protease cleavage site; a linker sequence; an amino acid sequence having an enzyme activity; and/ or an amino acid sequence providing for the fusion protein with binding affinity, such as a carbohydrate binding moiety

In an embodiment, the fusion protein comprises the amino acid sequence of the variant polypeptide and the at least one amino acid sequence providing a secretory signal sequence, said secretory signal sequence directing the fusion protein to secretory pathway of the host cell wherein the fusion protein is produced. In an embodiment, the fusion protein comprises a signal sequence which is advantageous in the translocation of the variant polypeptide within and out of the host cell. In an embodiment, the signal sequence is with or without a carrier polypeptide sequence.

In an embodiment, the fusion protein comprises an amino acid sequence which facilitates purification, such as His-tag or polyhistidine tag, wherein in the tag comprises a string of histidine residues. In an embodiment, the fusion protein comprises one or more sequences that control and direct the production of the variant polypeptide of fructanase.

In an embodiment, the fusion protein comprises a carbohydrate binding moiety (CBM) as a carrier polypeptide, which is optionally a fragment of another protein or enzyme derived from the same or different organism as the variant polypeptide. The signal sequence and carrier polypeptide may be derived from the same host cell, or alternatively derived from different hosts. In an embodiment, the fusion protein comprises a signal sequence originating from the same host cell wherein the fusion protein comprising the fructanase variant polypeptide is produced in. In an embodiment the signal sequence originates from *Trichoderma reesei,* wherein the fusion protein comprising the fructanase variant polypeptide is produced in. In another embodiment, the signal sequence originates from another organism than the host cell wherein the fusion protein comprising the fructanase variant polypeptide is produced in.

In an embodiment, the fusion protein comprises an amino acid sequence encoding a protease cleavage site. In an embodiment, the fusion protein comprises one or more amino acid linker sequences, linking two domains or parts of the fusion protein together.

In an embodiment, the fusion protein comprises an amino acid sequence having an enzyme activity, the said sequence with enzyme activity not being the same sequence as the amino acid sequence encoding the variant polypeptide with fructanase activity.

In an embodiment, the fusion protein comprises a carbohydrate binding moiety amino acid sequence, which provides the fusion protein with binding affinity. In an embodiment, the fusion protein comprises a stabilizer.

In an embodiment, an enzyme composition comprises the variant polypeptide or the fusion protein.

In an embodiment, an enzyme composition comprises at least the present variant polypeptide, and optionally a preservative, and/or a carrier. In an embodiment is provided the enzyme composition comprising the variant polypeptide or the fusion protein, and optionally one or more other polypeptides with enzymatic activity. In an embodiment, in addition to the variant polypeptide or the fusion protein, the enzyme composition may also comprise one or more enzymes having hydrolase activity.

In an embodiment the enzyme composition comprising the variant polypeptide of fructanase or the fusion protein and one or more additional enzymes is advantageous in providing a synergistic effect. Such an additional enzyme is desired when the present enzyme composition comprising variant polypeptide or the fusion protein is used in e.g., preparation of food products. Therefore, in an embodiment, the present fructanase enzyme is blended with other enzymes thereby obtaining the enzyme composition, to be used in baking, food, feed, and/or technical applications, for example.

Particularly advantageous synergistic enzymes that work with fructanase in food products are inulinases, levanases, endo-fructanases and invertases. Further particularly advantageous synergistic enzymes that work with fructanase in food applications, such as baking applications, are glucoamylases. In an embodiment, the enzyme composition comprising the variant polypeptide of fructanase or the fusion protein comprises an additional enzyme such as an enzyme liberating glucose or maltose. In an embodiment, the enzyme composition comprising the variant polypeptide of fructanase or the fusion protein comprises one or more enzymes selected from the group consisting of amylase, maltogenic amylase, beta amylase, aminopeptidase, carboxypeptidase, catalase, cellulloytic enzyme, chitinase, cutinase, cyclodextrin glycosyltransferase, deoxyribonuclease, esterase, glucan 1,4- alpha-maltotetrahydrolase, glucanase, galactanase, alpha-galactosidase, beta-galactosidase, glucoamylase, glucose oxidase, alpha-glucosidase, beta-glucosidase, haloperoxidase, hemicellulolytic enzyme, invertase, laccase, lipase, mannanase, mannosidase, oxidase, pectinolytic enzymes, peptidoglutaminase, peroxidase, phospholipase, phytase, polyphenoloxidase, proteolytic enzyme, ribonuclease, transglutaminase, xylanase, glucose isomerase, mannitol dehydrogenase and fructose dehydrogenase. In an embodiment, the enzyme composition comprising glucose isomerase, mannitol dehydrogenase and/or fructose dehydrogenase is advantageous with plant-based materials suitable for diet low in FODMAP, as such composition converts fructose into other molecules.

In an embodiment, the enzyme composition comprising the variant polypeptide of fructanase or the fusion protein comprises at least one additional enzyme, such as an enzyme used in baking, animal feed, hygiene product or in food, one or more other fructan degrading enzymes, sugar isomerases, sugar transferases and/or sugar dehydrogenases.

In an embodiment, the enzyme composition is a liquid composition comprising diluents such as water or buffer, and/or stabilizers, such as sorbitol, glycerol or propylene glycol, and/or preservatives, such as sodium benzoate, potassium sorbate or parabens.

In an embodiment, the enzyme composition is provided in the form of a liquid composition or a solid composition, or mixtures thereof. In an embodiment, the enzyme composition is provided in the form of a solution, dispersion, paste, flour, powder, granule, granulate, coated granulate, tablet, cake, crystal, crystal slurry, gel, or pellet.

The present variant polypeptide, the present fusion protein and/or the present enzyme composition is advantageous, as it has increased activity and/or productivity, when compared, for example, to a polypeptide according to SEQ ID NO: 12 or when compared to the wild type parent fructanase of the variant polypeptide. The present variant polypeptide, the present fusion protein and the present enzyme composition are advantageous, for example, for the degradation of fructan in grain materials, cereals, vegetables, and fruits.

The present variant polypeptide is preferably recombinantly produced non-naturally occurring protein. It can be prepared using generally known recombinant DNA technology. Briefly, the polynucleotide encoding a variant polypeptide is cloned and inserted into an expression vector, transformed into a host cell, and then expressed. Preferably, mutations are introduced into the coding sequence with codons preferred by the selected host strain. Methods for protein production by recombinant technology in different host systems are known in the art. Preferably, the variants are produced as extracellular proteins that are secreted into the culture medium wherein the host cell is cultured, and from which they can be easily recovered and isolated.

In an embodiment, a recombinant host cell comprises genetic elements that allow producing at least one variant polypeptide, or the fusion protein.

In an embodiment, the recombinant host cell is a microorganism, preferably selected from the group consisting of eukaryotic fungal cells, or prokaryotic bacterial cells, and yeast cells. In an embodiment, the host cell is selected from filamentous fungal cells such as *Trichoderma* or *Aspergillus*, or from gram-positive Bacilli such as *Bacillus.* In a preferable embodiment, the recombinant host cell is an eukaryotic fungal cell, preferably the host cell is a filamentous fungal cell, more preferably the host cell belongs to the genera *Trichoderma.* In preferable embodiment, the recombinant host cell is a filamentous fungal cell such as *Trichoderma* or *Trichoderma reesei*, *Aspergillus* or *Aspergillus oryzae* or A. *niger*, *Thermothelomyces* or *Thermothelomyces heterothallica*, *Myceliophthora* or *Myceliophthora thermophila*, *Humicola* or *Humicola insolens*, *Fusarium* or, *Fusarium venenatum.* In an embodiment, the recombinant host cell belongs to the order *Hypocreales.* In an embodiment, the recombinant host cell belongs to the family *Hypocreaceae.* In an embodiment, the recombinant host cell belongs to the genus *Trichoderma.*

In an embodiment, the recombinant host cell has an improved fructanase productivity when compared to a host cell comprising genetic elements that allow producing a polypeptide having the sequence of the wild type parent fructanase, or when compared to a host cell comprising genetic elements that allow producing a polypeptide having the sequence SEQ ID NO: 12. In an embodiment, the recombinant host cell comprises genetic elements that allow producing the at least one variant polypeptide or the fusion protein comprising the amino acid sequence of the variant polypeptide, wherein the recombinant host cell has an improved fructanase secretion when compared to a host cell comprising genetic elements that allow producing a polypeptide having the sequence of the wild type parent fructanase, or when compared to a host cell comprising genetic elements that allow producing a polypeptide having the sequence SEQ ID NO: 12.

In an embodiment, the relative fructanase productivity of the host cell is at least 1.5, preferably at least 2, more preferably at least 3, more preferably at least 4, even more preferably at least 6 fold higher, when compared to a to a host cell comprising genetic elements that allow producing a polypeptide according to SEQ ID NO: 12, or when compared to a host cell comprising the genetic elements that allow producing the wild type parent fructanase of the variant polypeptide. In a preferable embodiment, the relative fructanase productivity of the recombinant host cell is at least 10, preferably at least 20, more preferably at least 30 fold higher when compared to a host cell comprising genetic elements that allow producing a polypeptide according to SEQ ID NO: 12, or when compared to a host cell comprising the genetic elements that allow producing the wild type parent fructanase of the variant polypeptide. In an embodiment, the relative fructanase productivity of the recombinant host cell refers to the quantity of the fructanase variant polypeptide produced into the production medium.

The recombinant host cell can be used to produce the variant polypeptide of fructanase and to contain a polynucleotide encoding it. The selection of a specific recombinant host cell can be made in preparation of variants of fructanase with different properties. For example, a host cell can be selected, which provides post-translational modifications beneficial for stability or activity, or which facilitates processing of the variant polypeptide of fructanase produced in the host cell. The host cell can be operably linked to one or more control sequences to direct the production of the variant, and that make it possible to initiate the production of the present variant polypeptide by a stimulus, as is known in the field. In an embodiment the host cell is non-pathogenic. This is particularly advantageous when using the host cell, or the fructanase variant produced in it, for foodstuff, or feedstuff applications.

The present recombinant host cell is advantageous, as it has an increased productivity of the present variant polypeptide and/or the present fusion protein, when compared to, for example, a host cell comprising the polypeptide of the wild type parent fructanase of the variant polypeptide. The present recombinant host cell is advantageous, as it produces the present variant polypeptide and/or the present fusion protein having fructanase activity.

In an embodiment, the variant polypeptide and/or the fusion protein and/or the enzyme composition is used for degradation of fructan in plant-based material. In an embodiment, the use of the variant polypeptide and/or the fusion protein and/or the enzyme composition refers to method of using. In an embodiment, the use comprises bringing the variant polypeptide and/or the fusion protein and/or the enzyme composition in contact with fructan comprised by the plant-based material, thereby allowing the degradation of fructan comprised by said plant-based material into at least fructose. In an embodiment, the use comprises bringing the variant polypeptide and/or the fusion protein and/or the enzyme composition in contact with fructan comprised by the plant-based material, at reaction conditions allowing the degradation of fructan by the variant polypeptide.

In an embodiment, the fructan containing materials comprise:
cereals, such as wheat, spelt, rye and barley;
vegetables such as onions, shallots, garlic, leeks, artichoke, asparagus, beets, brussels sprouts, savoy cabbage, fennel, chicory, agave and snow peas;
nuts, such as cashews and pistachios;
pulses, such as kidney beans, black beans, mung means, navy beans, lima beans and split peas; and
fruits such as watermelon, nectarine, grapefruit, persimmon, pomegranate, plums, ripe bananas, dates, prunes and raisins.

In an embodiment, plant-based material is fructan containing material. In an embodiment, the variant polypeptide and/or the fusion protein and/or the enzyme composition can be used for the degradation of fructan in fructan containing materials. In an embodiment, the variant polypeptide and/or the fusion protein and/or the enzyme composition can be used for the degradation of fructan in grain and cereal containing materials, such as baked products including breads and cakes. In an embodiment, the variant polypeptide and/or the fusion protein and/or the enzyme composition can be used for the degradation of fructan in vegetable and/or fruit containing foods, such as vegetable containing foodstuff preparations.

In an embodiment, the fructan containing materials comprise processed food and/or convenience food.

In an embodiment, the use of the variant polypeptide and/or the fusion protein and/or the enzyme composition reduces fructan content of the fructan containing material at least 10 %, preferably at least 20 %, more preferably at least 30 %, more preferably at least 40 %, even more preferably at least 50 %, most preferably at least 60 % when compared to the fructan containing material without any fructanase enzyme. In an embodiment, the use of the variant polypeptide and/or the fusion protein and/or the enzyme composition reduces fructan content of the fructan containing material at least 20 %, at least 37 %, at least 63 % or at least 85 %, when compared to the fructan containing material without any fructanase enzyme.

In an embodiment, the variant polypeptide and/or the fusion protein and/or the enzyme composition is used to liberate fructose from fructan. In an embodiment, the use of the variant polypeptide and/or the fusion protein and/or the enzyme composition increases the fructose and glucose content of the fructan containing material, when compared to the fructan containing material without any fructanase enzyme. In an embodiment, the variant polypeptide and/or the fusion protein and/or the enzyme composition reduces the fructan content and increases the fructose content of the fructan containing material when compared to the fructan containing material without any fructanase enzyme.

In an embodiment, the use comprises a use of a combination of the present variant fructanase and at least one another enzyme. Particularly advantageous enzymes providing a synergistic effect at least in food products when used with the present fructanase polypeptide include: inulinases, levanases, endo-fructanases invertases, and glucoamylases. In an embodiment, the enzyme composition comprising the variant polypeptide of fructanase or the fusion protein is used in combination with an enzyme liberating glucose or maltose. In an embodiment, the enzyme composition comprising the variant polypeptide of fructanase or the fusion protein is used in combination with one or more enzymes selected from the group consisting of amylase, maltogenic amylase, beta amylase, aminopeptidase, carboxypeptidase, catalase, cellulolytic enzyme, chitinase, cutinase, cyclodextrin glycosyltransferase, deoxyribonuclease, esterase, glucan 1,4- alpha-maltotetrahydrolase, glucanase, galactanase, alpha-galactosidase, beta-galactosidase, glucoamylase, glucose oxidase, alpha-glucosidase, beta-glucosidase, haloperoxidase, hemicellulolytic enzyme, invertase, laccase, lipase, mannanase, mannosidase, oxidase, pectinolytic enzymes, peptidoglutaminase, peroxidase, phospholipase, phytase, polyphenoloxidase, proteolytic enzyme, ribonuclease, transglutaminase, xylanase, glucose isomerase, mannitol dehydrogenase and fructose dehydrogenase.

In an embodiment, the use of the variant polypeptide and/or the fusion protein and/or the enzyme composition comprises a use of a combination of the present variant fructanase and a glucoamylase for degradation of fructan in plant-based material, for keeping the ratio of fructose / glucose constant in the enzyme composition treated plant-based material. In an embodiment, the variant polypeptide and/or the fusion protein and/or the enzyme composition, is brought in contact with plant-based material together with glucoamylase, thereby degrading fructan and keeping the ratio of fructose / glucose constant in the plant-based material. In such an embodiment, the use, or the method wherein the variant polypeptide and a glucoamylase are used in combination, is a use in baking application, for example.

In an embodiment, the variant polypeptide and/or the fusion protein and/or the enzyme composition is used to degrade fructans in plant-based material, to obtain foodstuff suitable for a diet low in fermentable carbohydrates, known as FODMAP. In an embodiment, the use of the variant polypeptide and/or the fusion protein and/or the enzyme composition is especially beneficial in preparing products comprising plant-based materials suitable for diet low in FODMAP ("Fermentable, Oligo-, Di-, Monosaccharides, and Polyols"), the materials thereby having low fructan content.

In an embodiment, the variant polypeptide and/or the fusion protein and/or the enzyme composition is used to degrade fructans in plant-based material, to obtain fructose sirup, which fructose sirup can be used for further applications, such as alcohol fermentation. In such an embodiment, the use comprises adding the present variant polypeptide and/or the fusion protein and/or the enzyme composition to the application in question, and eventually recovering fructose sirup.

In an embodiment, the use or method of using comprises the use of the variant polypeptide, the fusion protein, and/or the enzyme composition in baking, manufacturing feedstuff, foodstuff, feed additive, a dietary supplement, a pharmaceutical, a detergent, cleaning product, hygiene products (such as mouth wash) processed food, animal feed (such as horse feed). In such embodiment, the use comprises adding the present variant polypeptide, the fusion protein, and/or the enzyme composition to the application in question, such as baking, and mixing.

In an embodiment, the variant polypeptide and/or the fusion protein and/or the enzyme composition is added to the plant-based material, such as flour or dough, in an amount of 10-10000 FRU (fuctanase units/ml) variant polypeptide per kg of the plant-based material. In an exemplary embodiment, the variant polypeptide is added in an amount of 100-2000 FRU, more preferably 500-1500 FRU of the variant polypeptide per kg of flour.

In an embodiment, the variant polypeptide and/or the fusion protein and/or the enzyme composition is used in preparation artificial sweeteners, such as low-calorie sweeteners (LCS). In an embodiment, the variant polypeptide and/or the fusion protein and/or the enzyme composition is used in preparation of fructose, which is beneficial at least because of low production costs. In an embodiment, the variant polypeptide and/or the fusion protein and/or the enzyme composition is used in manipulating fructan metabolism in plants.

Using the present variant polypeptide and/or the present fusion protein and/or the present enzyme composition is advantageous, as the variant polypeptide is capable of increasing degradation of fructan.

In an embodiment, a method of manufacturing the variant polypeptide or the fusion protein comprises: cultivating the recombinant host cell in conditions allowing production of the at least one variant polypeptide, or the fusion protein; and optionally recovering the variant polypeptide or the fusion protein.

In an embodiment of the method, the recombinant host cells are separated from the medium comprising the variant polypeptide and/or the fusion protein, an optionally, said medium is concentrated by removing water. The concentrate can be made, for example, by ultrafiltration, microfiltration, evaporation or any other suitable means. In an embodiment, the variant polypeptide and/or the fusion protein is separated from other proteins, optionally this is done by selective crystallization, precipitation, chromatographic methods, or any other suitable means.

The present method of manufacturing the variant polypeptide or the fusion protein is advantageous, as it allows production of the variant polypeptide or the fusion protein having increased fructanase productivity and/or improved fructan degrading performance, when compared to a polypeptide having the sequence SEQ ID NO: 12 or when compared to the wild type parent fructanase of the variant polypeptide.

In an embodiment, a premix for baking comprises the variant polypeptide and/or the fusion protein and/or the enzyme composition.

In an embodiment, the premix for baking comprises at least the variant polypeptide and/or the fusion protein and/or the enzyme composition, and one or more ingredients needed or suitable for baking. In an embodiment, the ingredients needed or suitable for baking include dry ingredients selected from one or more of flour, cereal grains or flakes, sugar, yeast or other leavening agents, fat or oil, salt, spices, flavorings, bakery ingredients, water, milk or milk alternative and eggs. In an embodiment, the ingredients needed or suitable for baking also include an improver for baking, comprising one or more ingredients from the group consisting of enzymes, wheat gluten, carriers (wheat gluten maltodextrin etc.), emulsifiers, and mono and diglycerides.

The present variant polypeptide, the present fusion protein and the present enzyme composition are advantageous in the premix for baking as the variant polypeptide is capable of increasing degradation of fructan in the premix and/or in the baking dough wherein the premix is mixed into. This is especially beneficial in preparing baked products suitable for LOW-FODMAP diet, having low fructan content.

In an embodiment, there is provided a food or foodstuff, comprising the variant polypeptide and/or the fusion protein and/or the enzyme composition. In an embodiment, the food or foodstuff, comprising the variant polypeptide and/or the fusion protein and/or the enzyme composition, is a mixture food product and/or processed food product, such as manufactured plant-based product/processed plant-based product, a supplement, an additive or mixtures thereof.

In an embodiment, there is provided a method of reducing fructan content in a baked product, the method comprising:
- providing ingredients for baking and mixing said ingredients together with the variant polypeptide and/or the fusion protein and/or the enzyme composition, thereby providing a dough, followed by proofing the dough, the fructan content in the dough being reduced while mixing and proofing; and
- baking the dough thereby providing the baked product having reduced fructan content.

In an embodiment, the method of reducing fructan content in a baked product comprises mixing the variant polypeptide and/or the fusion protein and/or the enzyme to the baking ingredients in an amount of 10-10000 FRU (fuctanase units/ml) of the variant polypeptide per kg of the plant-based material, such as flour, comprised by the baking ingredients. In an exemplary embodiment, the variant polypeptide is added in an amount of 500- 3000 FRU of the variant polypeptide per kg of flour. The dough produced with said method also has a reduced fructan content, the method thus also providing a method for reducing fructan content in a non-baked product such as dough.

In an embodiment, there is provided a baked product having reduced fructan content, the baked product comprising the variant polypeptide and/or the fusion protein and/or the enzyme composition, and wherein the fructan content is reduced compared to a baked product without any fructanase enzyme. In an embodiment, fructan content of the baked product is reduced at least 20 % or at least 37 %, when compared to a baked product which does not comprise any fructanase enzyme. In an embodiment, the baked product comprises the variant polypeptide and/or the fusion protein and/or the enzyme to the baking ingredients in an amount of 10-10000 FRU (fuctanase units/ml) variant polypeptide per kg of the plant-based material, such as flour, comprised by the baked product.

In an embodiment, there is provided an animal feed or feedstuff, comprising the variant polypeptide and/or the fusion protein and/or the enzyme composition. In an embodiment, the animal feed or feedstuff, comprising the variant polypeptide and/or the fusion protein and/or the enzyme composition, is a dry forage, roughage, pasture, silage, energy feed, protein supplement, mineral supplement, vitamin supplement, additive, or mixtures thereof. In an embodiment the feed is animal feed intended to be fed to animals, such as any compound feed or mixture. In another embodiment feed comprises grains such as maize, wheat, oats, barley, sorghum and rice; protein sources such as soybean meal, sunflower meal and canola meal; and/or one or more minerals.

In an embodiment, there is provided a detergent comprising the variant polypeptide and/or the fusion protein and/or the enzyme composition. In an embodiment, the detergent is provided in the form of a solution, dispersion, paste, powder, granule, granulate, coated granulate, tablet, cake, crystal, crystal slurry, gel, or pellet.

### EXAMPLES

### Example 1. Bio-IT search of fructanase enzymes

In order to identify suitable wild type fructanase candidates, homology-based BLAST searches were conducted. The GH32 domain area of the *Lactobacillus crispatus* fructosidase (the amino acids 408-890 of the SEQ ID NO: 2 disclosed in WO2017220864A1) was used as query in the BLAST search.

The BLAST search was conducted against several sequence databases, to get maximum coverage of the available sequences. The following databases were queried using command line BLAST: NCBI protein databases (nr, pdbaa), NCBI nucleotide databases (nt, env_nt), Uniprot protein database (including both SwissProt and TrEMBL), proprietary genomes of fungal and bacterial species, and patent sequence data via SequenceBase i.e. protein sequences from Thomson Reuters GENESEQ^{™}, USGENE^{®}, WOGENE.

The BLAST searches were conducted using a shell script in a Linux environment. The script goes through the databases and for each database conducts a batch BLAST search using all query proteins sequences simultaneously. E-value threshold of 1e-100 was used. The BLAST was requested to return 2000 best hits for each query. Otherwise, default parameters were used. The script then retrieved the DNA/AA sequence of matching hits. For BLAST hits from nucleotide databases a specially built script was used to process the xml-format BLAST output and extract the matching portion as an amino acid sequence.

After retrieval of the candidate sequences, a phylogenetic tree was constructed to get an understanding of the diversity among the retrieved sequences. The construction of the phylogenetic tree consisted of clustering of sequences to remove redundancy, followed by multiple sequence alignment, and building of the phylogenetic tree.

The protein sequences were clustered before the multiple sequence alignment and phylogenetic tree construction. This was done to remove the redundancy. To get an even coverage of the sequence space, the sequences were clustered to 95 % identity using the CD-HIT clustering program ((http://weizhongli-lab.org/cd-hit/). The selected candidates were aligned using MAFFT (https://mafft.cbrc.ip/aiignment/software/), a multiple sequence alignment program. A phylogenetic tree was built based on the multiple sequence alignment using FastTree (http://www.microbesoniine.org/fasttree/) algorithm. FastTree infers approximately-maximum-likelihood phylogenetic trees from alignments of nucleotide or protein sequences.

Candidate sequences for wild type fructosidase expression were selected from the branch containing the *Lactobacillus crispatus* fructosidase. This branch had 327 sequences of bacterial origin.

The database search of bacterial exo-fructosidases revealed potential fructosidases from several bacterial families and genus, including but not being limited to *Alteribacillus, Anaeromyces, Bacillus, Clostridium, Lachnospiraceae, Lactobacillus, Ligilactobacillus, Oscillibacter,* and *Streptococcus.* Altogether 15 sequences were selected for testing. Bacterial fructanase proteins have multiple putative domains in addition to the GH32 catalytic domain, as shown in the schematic picture of LFM37 fructanase polypeptide according to SEQ ID NO:12 of Figure 1.

### Example 2. Fructanase variant design

A set of variants were designed based on the mature wildtype *Streptococcus sobrinus W1703* fructanase (SEQ ID NO: 1), *Streptococcus parvulum* (SEQ ID NO:3), *Clostridium pasteurianum* DSM 525 (SEQ ID NO:5), *Lachnospiraceae bacterium* (SEQ ID NO:8), *Oscillibacter sp. fructosidase* (SEQ ID NO: 10), *Ligilactobacillus salivarius* (SEQ ID NO: 12), and *Ligilactobacillus equi* (SEQ ID NO:16 and SEQ ID NO: 18) for increased fructanase activity in *Trichoderma reesei.*

All the selected wild type fructanases, based on which the variants were designed, have a conserved N-glycosylation site at amino acid position corresponding to the N664 of SEQ ID NO: 12. Figure 2 shows an alignment of the wild type fructanase sequences (LFM30, LFM38, LFM29, LFM35, LFM40, LFM37 and LFM39) having a high sequence identity with each other upstream and downstream of the respective glycosylation site of conserved asparagine (N) at amino acid position corresponding to the N664 of SEQ ID NO: 12 (wild type LFM37). Therefore, this conserved N-glycosylation site was targeted when designing the variants comprising an amino acid mutation (substitution) preventing N-glycosylation the asparagine (N) of the variant polypeptide.

Different types of variant polypeptides were designed: variants with an N-terminal truncation, variants with a C-terminal truncation, variants with both N-terminal and C-terminal truncation, variants with an amino acid substitution preventing N-glycosylation of the conserved asparagine (N) at amino acid position corresponding to the N664 of SEQ ID NO: 12 (wild type LFM37), and variants with a combination of C-terminal truncation and said amino acid substitution. The SEQ ID numbers, Uniprot/ GB ID numbers and modifications done to the wild type molecules of the proteins are listed in Table 1. The sequence identity between the amino acids making up the GH32 domain (amino acids 374 - 850 ) of the wild type fructanase according to SEQ ID NO:12, and the corresponding amino acids of the indicated molecules are shown in the last column of Table 1.

**Table 1. List of fructanase variants designed and their sequence identifiers (SEQ ID NO).**

| Engineered sites (modifications) are presented with amino acid numbering corresponding to the respective mature wildtype (wt) fructanase protein sequence. The sequence identity percentage (%) is presented in reference to the amino acids 374 - 850 of SEQ ID NO: 12, i.e. the amino acids 374 - 850 of the molecule LFM37. | | | | |
|---|---|---|---|---|
| **Molecule** | **UniProt or GB ID** | **Modification** | **SEQ ID NO** | **SEQ identity** |
| LFM35 | U2KHV7_9STRE | none, is wt | 1 | 71 |
| LFM35GS | | T706A, C-terminal truncation STOP 1152 | 2 | |
| LFM38 | C8W893_ATOPD | none, is wt | 3 | 72 |
| LFM38GS | | N-terminal (START 151) and C-terminal truncation (STOP 1181), T734A | 4 | |
| LFM21 | A0A0H3J782_CLOPA | none, is wt | 5 | 49 |
| LFM21G | | T713A | 6 | |
| LFM21GS | | N-terminal (START 454) and C-terminal truncation (STOP 937), T583A | 7 | |
| LFM29 | MBR7045946.1 | none, is wt | 8 | 67 |
| LFM29G | | T607G | 9 | |
| LFM30 | MBQ9618515.1 | none, is wt | 10 | 66 |
| LFM30GS | | T905A, C-terminal truncation STOP 1350 | 11 | |
| LFM37 | A0A5C8H725_9LACO | none, is wt | 12 | 100 |
| LFM37G | | T666A | 13 | |
| LFM37S | | C-terminal truncation STOP 855 | 14 | |
| LFM37GS | | T666A, C-terminal truncation STOP 855 | 15 | |
| LFM39 | WP_051527988.1 | none, is wt | 16 | 76 |
| LFM39GS | | T503A, C-terminal truncation STOP 689 | 17 | |
| LFM40 | WP_023859329.1 | none, is wt | 18 | 69 |
| LFM40GS | | T688A, C-terminal truncation STOP 873 | 19 | |

### Example 3. Production of wild type (wt) fructanases and respective variants thereof

Standard molecular biology methods were used in the isolation and enzyme treatments of DNA (e.g., isolation of plasmid DNA, digestion of DNA to produce DNA fragments), in *E. coli* transformations, sequencing etc. The basic laboratory methods used were either as described by the enzyme, reagent or kit manufacturer or as described in the standard molecular biology handbooks, e.g., in Sambrook & Russell, 2001 or as described in the following examples.

Expression plasmids were constructed for production of wildtype fructanases and fructanase variants. The genes of interest (GOls) encoding the designed fructanase variants (Table 1) were ordered as synthetic genes using codons optimised for expression in *T. reesei.* The fructanases were expressed with *T. reesei* cbh1 (cel7A) promoter using an N-terminal carrier polypeptide (CBM and linker) encoding sequence from *T. reesei* cbh2 (cel6A). A Kex2 protease cleavage site was included between the carrier polypeptide and the fructanase as described in (Paloheimo et al., 2003). Transcription was terminated using *T.reesei* cbh2 (cel6A) terminator, and a synthetic amdS marker gene was used for selecting transformants. In addition, the fructanase expression constructions contain cbh1 (cel7A) 5' and 3' flanking regions for optional targeting of the expression vector into the cbh1 (cel7A) locus. A schematic picture of the expression plasmids is presented in Figure 3.

*Clostridium pasteurianum* fructanase (SEQ ID NO: 5 - 7) was expressed from *T. reesei* cbh1 (cel7A) promoter. Transcription was terminated using *T.reesei* cbh2 (cel6A) terminator, and a synthetic amdS marker gene was used for selecting transformants. In addition, the fructanase expression constructions contain cbh1 (cel7A) 5' and 3' flanking regions for optional targeting of the expression vector into the cbh1 (cel7A) locus.

Circular expression plasmids, as disclosed in Figure 3, were transformed in *T. reesei* protoplasts. The transformants were selected on plates containing acetamide as the sole nitrogen source. The *T. reesei* host strain used lacks the four main endogenous cellulases: CBHI/Cel7A, CBHII/Cel6A, EGI/Cel7B and EGII/Cel5A. The transformations were performed according to (Penttilä et al., 1987), with the modifications described in (Karhunen et al., 1993). Alternatively, CR!SPR-CAS technology can be used in transformations.

Transformants were purified on selection plates through single conidia prior to sporulating them on potato dextrose agar (PDA). The fructanase production of the transformants was analyzed from the culture supernatants of shake flask cultivations (50 mL). Transformants were grown for 7 days at 30 °C, 250 rpm in a complex cellulase-inducing medium (Joutsjoki et al., 1993) buffered with 5 % (w/v) KH₂PO₄ at pH 6.0.

Production of the recombinant protein was detected from the culture supernatant by SDS-polyacrylamide gel electrophoresis (not shown). A distinct high-molecular weight band was detected in the samples of all the fructanase supernatants analysed, except for LFM30. Based on SDS-PAGE analysis, the variant fructanases, i.e., variants with prevented N-glycosylation in the asparagine at position corresponding amino acid position N664 of SEQ ID 12 (LFM37), and/or variants with a C-terminal truncation have an increased fructanase production, when compared to the wildtype fructanases. Hence, a C-terminal truncation of the wildtype fructanases or de-glycosylation of the asparagine at an amino acid position corresponding amino acid position N664 of SEQ ID 12 (LFM37), resulted into higher fructanase productivity. The fructanase production level of the wild type fructanases and their respective variants, as measured from the SDS-PAGE, are shown in table 2.

**Table 2. The relative fructanase production level of the fructanase variants produced in T. reesei, when compared to the respective wild type fructanases production in T. reesei. All samples were analysed in duplicates. The indicated symbols of the production level refer to: (-): no production; (+): production detected; (++): production increased; (+++): production strongly increased.**

| Sample | Production level (SDS-PAGE) |
|---|---|
| LFM35 | + |
| LFM35GS | ++ |
| LFM38 | - |
| LFM38GS | ++ |
| LFM21 | + |
| LFM21G | - |
| LFM21GS | + |
| LFM29 | + |
| LFM29G | ++ |
| LFM30 | - |
| LFM30GS | ++ |
| LFM37 | ++ |
| LFM37G | ++ |
| LFM37S | +++ |
| LFM37GS | +++ |

### Example 4. Analysis of wild type fructanases and respective variants

The enzyme activity of the recombinant fructanases was measured from the culture supernatant as the release of reducing sugars (i.e. fructose residues) from inulin using Fluent^{®} automation workstation (Tecan Group Ltd, Männedorf, Switzerland).

The samples were diluted in a 0.05 M sodium acetate buffer pH 5.0, and the substrate, inulin from chicory (Sigma No. I2255), was dissolved in the same buffer. A substrate concentration of 0.25 % (w/v) was used. Sample dilution (25 µL) was mixed with the preheated substrate (225 µL) in the DeepWell plate and incubated at 37 °C, for 15 min. After 15 min incubation, 375 µL dinitrosalicylic reagent (10 g of 3,5-dinitrosalicylic acid, 16 g of NaOH, 300 g of potassium sodium tartrate tetrahydrate dissolved in distilled water and filled-in to 1 liter) was added, and the reaction was stopped by boiling the mixture for 8 min. Samples were cooled for 5 min at 4 °C and thereafter for 3 min at 20 °C. After the cooling step 200 µL of each reaction mixture was transferred into a 96 well microplate. The absorbance was measured at 540 nm in a microplate spectrophotometer (Tecan, Spark).

A calibration curve was obtained using fructose as the standard (7.5, 10, 14, 20, 30, 40 and 50 µmol/mL). Fructose standards were treated identically to the samples. One enzymatic unit was defined as an amount of the enzyme which produces 1 µmol of reducing sugars per mL. The amount of released fructose is calculated from the calibration curve. A relative fructanase activity of the measured variants was calculated as fold change in reference to the respective wildtype fructanase enzyme in question.

The results showing the relative fructanase activity of the fructanase variants are shown in Table 3. The relative fructanase activity measured from the culture supernatant of the wildtype fructanases (LFM35/SEQ ID NO: 1; LFM38/SEQ ID NO: 3; LFM21/SEQ ID NO: 5; LFM29/SEQ ID NO: 8) and LFM21G (SEQ ID NO: 6) with amino acid exchange T713A were at the level of the host cell background i.e., no fructanase activity was detected. Increased relative fructanase enzyme activity was obtained with the full-length fructanase variants with amino acid substitutions resulting in no N-glycosylation (LFM29G, LFM37G) and with fructanase variant with C-terminal truncation (LFM37S). A combination of amino acid substitution resulting in no N-glycosylation and a C-terminal truncation leads in all cases to a substantial increase of relative fructanase activity (LFM35GS, LFM38GS, LFM30GS, LFM37GS, LFM40GS). The results indicate that amino acid substitutions resulting in blocking (or prevention) of N-glycosylation leads to production of an active fructanase in a host cell which is capable of N-glycosylation. A combination of C-terminal and/or N-terminal truncation with the amino acid substitution inhibiting N-glycosylation resulted in further increased fructanase activity, when compared to variants having the substitution inhibiting N-glycosylation or truncation alone (see Table 3).

**Table 3. The relative fructanase activity of the fructanase variants produced in T. reesei, when compared to the respective wild type fructanases produced in T. reesei. The relative fructanase activity was measured from the shake flask culture supernatants as FRU/mL (Inulin). All samples were analysed in duplicates.**

| Fructanase molecule | SEQ ID NO | Relative fructanase activity |
|---|---|---|
| Empty host | none | 1 |
| LFM35 | 1 | 1 |
| LFM35GS | 2 | 43 |
| LFM38 | 3 | 1 |
| LFM38GS | 4 | 108 |
| LFM21 | 5 | 1 |
| LFM21G | 6 | 1 |
| LFM29 | 8 | 1 |
| LFM29G | 9 | 44 |
| LFM30 | 10 | 1 |
| LFM30GS | 11 | 449 |
| LFM37 | 12 | 255 |
| LFM37G | 13 | 360 |
| LFM37S | 14 | 363 |
| LFM37GS | 15 | 753 |
| LFM40 | 18 | 1 |
| LFM40GS | 19 | 93 |

### Example 5. Characterisation of wild type fructanases and respective variants

The pH optimum of the recombinant fructanase variants was determined in the universal Mcllvaine's buffer within a pH range of 4.0 - 8.0, using inulin as a substrate. The reaction was carried out at 40 °C for 10 min.

Substrate specificity of the fructanase variants was determined by high-performance liquid chromatography (HPLC). Two substrates, inulin from chicory (Sigma No. I2255) and Timothy grass levan (P-LEVAN, Magazyme), were used in the analysis. One mL of 2 % (w/v) substrate solution was equilibrated at 40 °C for 5 min, followed by addition of 20 µL of the sample (diluted in 50 mM acetate buffer at pH 5.0). Reaction mixtures were incubated at 40 °C for 10 min and thereafter enzymatic reaction was terminated by placing the tubes in a boiling water bath for 5 min. The samples were then cooled to room temperature and filtered through 0.2 µm membrane before HPLC analysis.

Hydrolytic reaction products (liberated fructose) from the reaction mixtures were analysed and quantified by HPLC as follows. A Dionex DC5000+ ion chromatography system equipped with the ED40 electrochemical detector and Dionex CarboPac PA1 analytical column was used for the analysis. A sample (25 µL) of each reaction product was applied into the column, followed by elution with two solvents: 0.1 M aqueous sodium hydroxide (A) and 0.1 M aqueous sodium hydroxide containing 0.5 M sodium acetate (B). Elution of the reaction products was started with 0.1 M sodium hydroxide solution for 10 minutes at a flow rate of 0.5 mL/minute. Thereafter, a linear gradient of 0 - 90 % solvent B in 0.1 M sodium hydroxide was applied for 30 min. Detected fructose peak area was calculated using Chromelon 7.3 software (Thermo Fisher Scientific Inc.) and used for determination of inulinase / levanase activity ratio. The results indicate that some of the wt fructanases (LFM21, LFM29, LFM30, LFM37) and variants thereof are more active against levan than inulin, whereas some of the wt fructanases (LFM35, LFM38) and variants thereof are more active against inulin than levan (see Table 4).

**Table 4. shows pH optimum, temperature optimum, as well as enzyme substrate specificity of fructanase variants. All samples were analysed in duplicates. The minus symbol (-) in the table 4 indicates the molecule was not expressed.**

| Sample | pH-opt | Tm-opt (°C) | Substrate specificity | Inulin/ Levan |
|---|---|---|---|---|
| LFM35 | - | - | | |
| LFM35GS | 6 | 50 | Levan | 0.4 |
| LFM38 | - | - | | |
| LFM38GS | 6 | 40 | Levan | 0.8 |
| LFM21 | - | - | | |
| LFM21G | - | - | | |
| LFM21GS | 6 | 40 | Inulin | 1.2 |
| LFM29 | - | - | | |
| LFM29G | 6 | 40 | Inulin | 1.8 |
| LFM30 | - | - | | |
| LFM30GS | 6 | 50 | Inulin | 1.5 |
| LFM37 | 5 | 50 | Inulin | |
| LFM37G | 5 | 50 | Inulin | 2.4 |
| LFM37S | 5 | 50 | Inulin | |
| LFM37GS | 5 | 50 | Inulin | |

### Example 6. Baking trials with fructanase variants

Breads containing different fructanase variants, each dosed to 1000 FRU/kg flour, and reference breads without fructanase enzymes were made according to following dough recipe:
1 kg rye flour T1150, 860 g water, 15 g salt, 11 g dough acidifier (Boerol from CSM Ingredients Germany) and 20 g fresh yeast.

After 7 minutes of mixing, 25 minutes of resting, 40 minutes of fermentation at 34°C, the dough was baked to bread at 230°C for 55 minutes. Thereafter, bread was left at room temperature to cool down over night.

Then bread was cut into slices and the breadcrust removed. From these slices breadcrumbs were cut with a sharp knife as small as possible and weighted into 22x200 mm glass tubes with screw caps. To each tube was added 500 mg breadcrumbs and 25 ml of pure water. The tubes were loosely capped and placed into a boiling water bath. After 5 minutes, the tubes were taken out of the water bath and the tube caps were tightened and the contents were vigorously mixed by inversion and vortexing the tubes. Then the tubes were returned to the boiling water bath for further 5 minutes. After a total of 10 minutes in the water bath, the tube contents were again mixed by inversion and vortexing. The tubes were allowed to cool down to room temperature. Approximately 2 ml of the tube contents were transferred into 2 ml plastic tubes and centrifuged at 13000 rpm for 10 minutes. Supernatants were obtained from each tube and these were then stored frozen. After thawing, the supernatant samples were vortexed and then centrifuged again at 13000 rpm for 10 minutes at room temperature. The Megazymes kits K-FRUC and K-FRUGL were used to measure in duplicates the concentration of fructan, fructose and glucose. The results are shown in Table 5.

**Table 5. Concentration of fructan, fructose and glucose in breads, reported in grams (g) per 100 grams of bread. Breads were baked with different fructanase variants (LFM38G, LFM21GS, LFM29G, LFM30GS, LFM37G). A reference bread without fructanase enzyme was made as a control bread and labelled "no fructanase".**

| | **Fructan** | **Fructose** | **Glucose** |
|---|---|---|---|
| No fructanase | 0.86 | 0.73 | 0.27 |
| LFM38G | 0.13 | 1.48 | 0.39 |
| LFM21GS | 0.82 | 0.82 | 0.29 |
| LFM29G | 0.32 | 1.27 | 0.35 |
| LFM30GS | 0.69 | 0.96 | 0.31 |
| LFM37G | 0.54 | 1.08 | 0.32 |

Using the values in the table 5, the net change due to fructanase enzyme addition in fructan, fructose and glucose concentration was calculated and reported in the table 6.

**Table 6. Change (Δ) in fructan, fructose and glucose concentration in breads, reported in grams per 100 grams of bread. Breads were baked with different fructanase variants (LFM38G, LFM21GS, LFM29G, LFM30GS, LFM37G).**

| | **Δ Fructan** | **Δ Fructose** | **Δ Glucose** |
|---|---|---|---|
| LFM38G | - 0.73 | 0.75 | 0.11 |
| LFM21GS | - 0.04 | 0.09 | 0.02 |
| LFM29G | - 0.54 | 0.54 | 0.07 |
| LFM30GS | - 0.17 | 0.23 | 0.03 |
| LFM37G | - 0.32 | 0.35 | 0.05 |

All tested fructanase variants showed a decrease in fructan content and a concomitant increase in fructose content in bread, when compared to bread sample baked without any fructanase enzyme. All tested fructanase variants showed also a small increase in glucose content in bread, when compared to bread sample baked without any fructanase enzyme. All tested fructanase variants had fructanase activity during bread making in the dough and caused the desired decrease in fructan content of bread. These results indicate, the present fructanase variants are efficient in degradation of fructan in plant-based material, such as rye flour, in baking applications.

Various embodiments have been presented. It should be appreciated that in this document, words comprise, include, and contain are each used as open-ended expressions with no intended exclusivity.

The foregoing description has provided by way of non-limiting examples of particular implementations and embodiments a full and informative description of the best mode presently contemplated by the inventors for carrying out the invention. It is however clear to a person skilled in the art that the invention is not restricted to details of the embodiments presented in the foregoing, but that it can be implemented in other embodiments using equivalent means or in different combinations of embodiments without deviating from the characteristics of the invention.

Furthermore, some of the features of the afore-disclosed example embodiments may be used to advantage without the corresponding use of other features. As such, the foregoing description shall be considered as merely illustrative of the principles of the present invention, and not in limitation thereof. Hence, the scope of the invention is only restricted by the appended patent claims.

### References

Joutsjoki VV, Torkkeli TK, and Nevalainen KMH. (1993) Transformation of Trichoderma reesei with the Hormoconis resinae glucoamylase P (gamP) gene: production of a heterologous glucoamylase by Trichoderma reesei. Curr. Genet. 24: 223-228.
Karhunen T, Mäntylä A, Nevalainen KMH and Suominen P. 1993. High frequency one-step gene replacement in Trichoderma reesei. I. Endoglucanase I over poroduction. Mol. Gen. Genet. 241:515 - 522.
Paloheimo M, Mäntylä A, Kallio J, and Suominen P. (2003) Highyield production of a bacterial xylanase in the filamentous fungus Trichoderma reesei requires a carrier polypeptide with an intact domain structure. Appl. Env. Microbiol. 69: 7073-7082.
Penttilä M., Nevalainen H., Rättö M, Salminen E and Knowles J. 1987. A versatile transformation system for the cellulolytic filamentous fungus Trichoderma reesei. Gene 61:155-164.
Sambrook J, and Russell DW. (2001) Molecular cloning, a laboratory manual. Cold Spring Harbor Laboratory, New York, US.
Sievers F., Wilm A., Dineen D., Gibson T.J., Karplus K., Li W., Lopez R., McWilliam H., Remmert M., Söding J., Thompson J.D. and Higgins D.G. 2011. Fast, scalable generation of high-quality protein multiple sequence alignments using Clustal Omega. Mol. Syst. Biol. 7:539.

## Claims

1. A variant polypeptide of a wild type fructanase, having:
fructanase activity;
an amino acid sequence having at least 65 % amino acid sequence identity with the amino acids 374 - 850 of SEQ ID NO: 12; and
at least one substitution, insertion, or deletion of an amino acid, the at least one substitution, insertion or deletion preventing N-glycosylation of at least one amino acid when expressed in a host cell capable of N-glycosylation, and/or
at least 50 amino acids deleted when compared to the wild type parent fructanase of the variant polypeptide.

2. The variant polypeptide of claim 1, wherein the amino acid sequence has the at least one substitution, insertion or deletion of an amino acid, wherein the at least one amino acid substitution, insertion or deletion is in the amino acid region of the variant polypeptide corresponding to the amino acids 374 - 850 of SEQ ID NO: 12.

3. The variant polypeptide of claim 1 or 2, wherein the amino acid sequence has the at least one substitution, insertion or deletion of an amino acid, wherein the at least one amino acid substitution, insertion or deletion disrupts a consensus sequence for N-glycosylation FNGT(x1)NL(x2)L, where (x1) is Y or F, and (x2) is N or F.

4. The variant polypeptide of any preceding claim, wherein the amino acid sequence has the at least one substitution, insertion or deletion of an amino acid, and the at least one amino acid substitution, insertion or deletion comprises at least one amino acid substitution or deletion at amino acid position corresponding to any of the amino acid positions 664- 666 of the SEQ ID NO: 12.

5. The variant polypeptide of any preceding claim, wherein the amino acid sequence comprises at least the amino acids corresponding to the amino acids 374 - 850 of the SEQ ID NO: 12.

6. The variant polypeptide of any preceding claim, having the at least 50 amino acids deleted from its amino acid sequence, wherein the at least 50 amino acids, or at least 100 amino acids are deleted in amino acid positions corresponding to the amino acid positions 1 - 373, and/or positions 851 - 973 of SEQ ID NO: 12, preferably corresponding to the amino acid positions 851 - 973 of SEQ ID NO: 12.

7. The variant polypeptide of any preceding claim, wherein the amino acid sequence has at least 70 %, preferably at least 75 %, more preferably at least 80 %, even more preferably at least 85 % amino acid sequence identity with the amino acids 374 - 850 of SEQ ID NO: 12.

8. The variant polypeptide of any preceding claim, wherein the variant polypeptide is of bacterial origin, preferably originating from the family *Lactobacillaceae, Oscillospiraceae, Lachnospiraceae, Clostridiaceae, Streptococcacea, Atopobiaceae, Bacillaceae or Neocallimastigaceae* wild type bacterial fructanases, more preferably originating from the genus *Ligilactobacillus, Oscillibacter, Clostridium, Streptococcus, Lancefieldella, Lactobacillus, Bacillus, Alteribacillus* or *Anaeromyces* wild type bacterial fructanases, most preferably from the species *Ligilactobacillus salivarius* wild type fructanase.

9. The variant polypeptide of any preceding claim, having an increased fructanase productivity and/or improved fructan degrading performance, when compared to a polypeptide having the sequence SEQ ID NO: 12 or when compared to a wild type parent fructanase of the variant polypeptide.

10. A fusion protein, comprising the amino acid sequence of the variant polypeptide according to any one of claims 1-9, and at least one:
an amino acid sequence providing a secretory signal sequence;
an amino acid sequence which facilitates purification, such as an affinity tag or His-tag;
an amino acid sequence controlling and enhancing the production of the fusion protein, such as a carrier polypeptide;
an amino acid sequence encoding a protease cleavage site;
a linker sequence;
an amino acid sequence having an enzyme activity; and/or
an amino acid sequence providing for the fusion protein with binding affinity, such as a carbohydrate binding moiety.

11. An enzyme composition comprising the variant polypeptide of any one of claims 1-9 or the fusion protein of claim 10.

12. A recombinant host cell comprising genetic elements that allow producing at least one variant polypeptide of any one of claims 1-9, or the fusion protein of claim 10.

13. Use of the variant polypeptide of any one of claims 1-9, and/or the fusion protein of claim 10 and/or the enzyme composition of claim 11, for degradation of fructan in plant-based material.

14. A method of manufacturing the variant polypeptide of any one of claims 1-9, or the fusion protein of claim 10, comprising:
cultivating the recombinant host cell of claim 12 in conditions allowing production of the at least one variant polypeptide of any one of claims 1-9, or the fusion protein of claim 10; and
optionally recovering the variant polypeptide or the fusion protein.

15. A premix for baking, comprising the variant polypeptide of any one of claims 1-9, and/or the fusion protein of claim 10 and/or the enzyme composition of claim 11.
